# EUROPEAN PATENT SPECIFICATION

(11) **EP 3 882 627 B1**
(45) Date of publication and mention of the grant of the patent: **10.07.2024**
(21) Application number: 19883598.5
(22) Date of filing: 13.11.2019
(51) Int. Cl.: G01N 33/48, A01N 1/02, G01N 33/68

(54) **CONTAINER AND METHOD FOR STORING, PRETREATING, AND ANALYZING BIOMATERIAL**
BEHÄLTER UND VERFAHREN ZUM LAGERN, VORBEHANDELN UND ANALYSIEREN VON BIOMATERIAL
RÉCIPIENT ET PROCÉDÉ DE STOCKAGE, DE PRÉTRAITEMENT ET D'ANALYSE DE MATÉRIEL BIOLOGIQUE

(30) Priority: 14.11.2018 JP 2018213960
(43) Date of publication of application: 22.09.2021
(73) Proprietor: Nissan Chemical Corporation, Tokyo 103-6119 (JP)
(72) Inventor: OTSUKA, Keiichiro, Saitama 349-0294 (JP); HAMADA, Satoshi, Chiba 274-8507 (JP); SHOJI, Takeaki, Chiba 274-8507 (JP); NAGAE, Kei, Chiba 274-8507 (JP); NAKAJIMA, Hiroyuki, Saitama 349-0294 (JP); SUZUKI, Kohei, Chiba 274-0052 (JP); MATOBA, Kazutaka, Saitama 349-0294 (JP)
(74) Representative: dompatent von Kreisler Selting Werner - Partnerschaft von Patent- und Rechtsanwälten mbB
(86) International application number: PCT/JP2019/044605
(87) International publication number: WO 2020/100957

(56) References cited:
- EP-A1- 3 467 095
- WO-A1-00/39582
- WO-A1-2014/196650
- WO-A1-2017/217336
- WO-A1-2019/176515
- JP-A- 2006 158 961
- JP-A- 2007 063 459
- JP-A- 2008 191 067
- JP-A- 2009 031 121
- JP-A- 2018 169 349
- NL-A- 7 603 497
- US-A1- 2010 096 327

## Description

### TECHNICAL FIELD

The present invention relates to use of a container for storing a biomaterial for reducing sample loss of a biomarker(s) contained in the biomaterial, and in particular, it relates to use of a container for storing a biomaterial which suppresses adhesion of a minute amount of the biomarker(s) contained in the biomaterial and is provided with a coating which reduces sample loss.

### BACKGROUND ART

In the biological substances such as peptides, proteins, endogenous metabolites or genes, etc., contained in a biomaterial such as blood and urine, those which correlate with changes in diseases and response to treatment, and serve as indicators are called as biomarkers. In the biomarkers, there has been known diagnostic markers, prognostic markers, predictive markers, monitoring markers, safety markers, and the like, has been known depending on the purpose. In recent years, particularly in the diagnosis and treatment of cancer, attempts have been made as one of indicators of the presence or progression of cancer and the effect of treatment, by measuring an amount of tumor markers.

Biomarkers contained in biomaterials are generally in a minute amount, and the amount increases or decreases in correlation with the presence and progression of the disease and response to treatment. Accordingly, in order to properly evaluate the biomarkers, it is necessary to measure with accuracy and highly precision using an apparatus capable of enabling highly sensitive analysis of a minute amount component like a liquid chromatography/mass spectrometer (LC-MS). For such measurements, in addition to the requirement of the apparatus and method for measuring a biomaterial, the requirement relating to the quality of the material to be measured is also important. For example, a biomaterial collected from a subject person is generally stored in a container for storage such as a tube made of glass or plastic until it is applied to the measurement, but there is a problem that proteins and endogenous metabolites that are biomarkers are adsorbed on the inner surface of the container to cause sample loss, whereby it affects proper evaluation of the biomarkers.

In order to solve such a problem, it has heretofore been proposed a container for storing a biomaterial by modifying the inner surface thereof hydrophilic by plasma treatment or corona discharge treatment, or by surface treatment with a photocrosslinkable superhydrophilic polymer, whereby adsorption of the proteins and endogenous metabolites including the biomarkers is suppressed to reduce sample loss. For example, US20100096327 discloses a composite membrane comprising a support membrane and a dense polymer layer attached to at least a portion of the outer surface of said support, wherein the polymer layer comprises surface quaternary phosphonium or ammonium function groups. EP3467095 discloses a cell culture vessel having a surface coated with a copolymer having a function of inhibiting adhesion of cells.

The present inventors have paid attention to a polymer having a phosphoric acid ester group, which is expected as a coating material having an ability to suppress adhesion of various biological substances, and repeated studies. As a result, they have reported that a coating agent containing a copolymer which contains specific anionic group and cationic group can be firmly fixed without selecting the kind of the substrate, and after fixing, it becomes a coating film excellent in resistance to an aqueous solvent, and shows excellent adhesion suppressing ability to biological substances (for example, platelets, fibrinogen, etc.) (for example, see WO2014196650 and WO2016093293). Also, they have reported that a cell culture container characterized in that it is coated with the same coating agent is excellent in adhesion suppressing ability to cells and also excellent in resistance to solvents and radiation (for example, see WO2014196652).

### SUMMARY OF THE INVENTION

### PROBLEMS TO BE SOLVED BY THE INVENTION

In a coating applied to a container for storing a biomaterial, it is desirable not only to have an ability to suppress cell adhesion and resistance to aqueous solvents and radiation, but also to have an ability to suppress adhesion of proteins and endogenous metabolites, etc., which are a minute amount of a biomarker(s) contained in the biomaterial, from the viewpoint of reducing sample loss and enabling analysis with accuracy and highly precision. However, it has not been reported a container for storing a biomaterial provided with a coating having both of these.

### MEANS TO SOLVE THE PROBLEMS

The present inventors have found that a container for storing a biomaterial providing with a coating containing a copolymer which contains a specific anion structure, a specific cation structure, and optionally a specific hydrophobic structure on at least a part of a surface of the container has not only an excellent ability to suppress cell adhesion and resistance to aqueous solvents and radiation, but also has an excellent ability to suppress adhesion of a minute amount of a biomarker(s) contained in the biomaterial, can reduce sample loss, and consequently the biomarker(s) can be appropriately evaluated, whereby they have completed the present invention.

The present invention provides use of a container for storing a biomaterial for reducing sample loss of a biomarker(s) contained in the biomaterial defined in independent claim 1, an analytical method of a fragmented protein and/or peptide defined in independent claim 7 as well as a method for measuring an absolute amount(s) of a biomarker(s) in a solution defined in independent claim 8. Preferred embodiments are reflected in the dependent claims.

The present invention is as follows.
[1] Use of a container for storing a biomaterial for reducing sample loss of a biomarker(s) contained in the biomaterial, which comprises a coating containing a copolymer which contains
   a recurring unit which contains a group represented by the following formula (a) and a recurring unit which contains a group represented by the following formula (b): 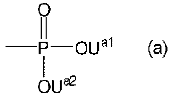 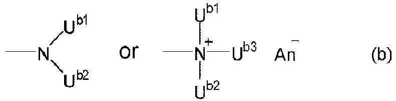 (wherein
   U^{a1}, U^{a2}, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion)
   provided with at least a part of a surface of the container.
[2] Use described in [1], wherein the copolymer further comprises a recurring unit which contains a group represented by the following formula (c):

   -R^{c} (c)

   [wherein,
   R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (here, the aryl portion may be substituted by a linear or branched alkyl group having 1 to 5 carbon atoms which may be substituted by a halogen atom(s))].
[3] Use described in [1] or [2], wherein the biomarker(s) is/are an antibody or amyloid.
[4] Use of a container for storing a peptide which comprises a coating containing a copolymer which contains
   a recurring unit which contains a group represented by the following formula (a) and a recurring unit which contains a group represented by the following formula (b): 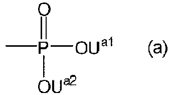 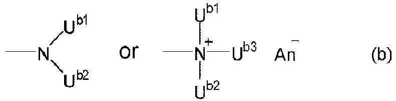 (wherein
   U^{a1}, U^{a2}, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion)
   provided with at least a part of a surface of the container.
[5] Use of a container for storing a peptide described in [4], wherein the peptide is contained in a biomaterial applied to mass analysis.
[6] Use of a container for fragmentation treatment of a protein and/or a peptide, which comprises a coating containing a copolymer which contains a recurring unit which contains a group represented by the following formula (a) and a recurring unit which contains a group represented by the following formula (b): 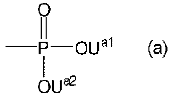 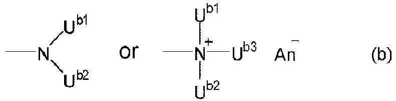 (wherein
   U^{a1}, U^{a2}, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion)
   provided with at least a part of a surface of the container.
[7] An analytical method of a fragmented protein and/or peptide, which comprises
   preparing a sample containing a protein and/or a peptide which is/are an analytical obj ect(s),
   subjecting the protein and/or the peptide in the sample to fragmentation treatment in a container provided with a coating containing a copolymer which contains a recurring unit which contains a group represented by the following formula (a) and a recurring unit which contains a group represented by the following formula (b): 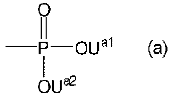 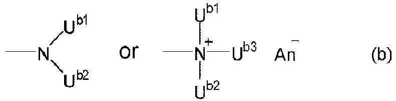 (wherein
      U^{a1}, U^{a2}, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion)
      at least a part of a surface of the container, and
   applying the fragmented protein and/or peptide in the sample to a measurement apparatus.
[8] A method for measuring an absolute amount(s) of a biomarker(s) in a solution, which comprises:
   a step of containing and/or storing a solution containing a biomarker(s) contained in a biomaterial in a container for storing a biomaterial having a coating which contains a copolymer containing a recurring unit which contains a group represented by the following formula (a) and a recurring unit which contains a group represented by the following formula (b): 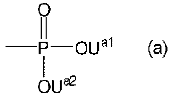 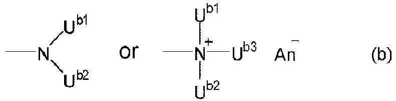 (wherein
   U^{a1}, U^{a2}, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion)
   on at least a part of a surface of the container, and then, a step of measuring an absolute amount(s) of the biomarker(s).
[9] The method described in [7], wherein the biomarker(s) is/are an antibody or amyloid.

### EFFECTS OF THE INVENTION

In the container for storing a biomaterial of the present invention, by providing a coating having an excellent ability to suppress cell adhesion to proteins and endogenous metabolites, etc., which are a minute amount of a biomarker(s) contained in the biomaterial and enables reduction of sample loss, in particular, a coating containing a copolymer which contains an anionic group represented by the formula (a), a cationic group represented by the formula (b), and optionally a hydrophobic group represented by the formula (c) on at least a part of a surface of the container, preferably by coating on the entire surface of the container, adhesion of the minute amount of the biomarker(s) contained in the biomaterial to the container can be suppressed. Also, by forming an ionic bond (ion complex) by the cationic group and anionic group in the coating, it can be adhered without selecting the kind of the substrate of the container such as glass, fiber, inorganic particles or resin (synthetic resin and natural resin), etc., and after fixing, it becomes a coating excellent in resistance to aqueous solvents (water, phosphate buffered saline (PBS), an alcohol, etc.). That is, according to the present invention, use of an excellent container for storing a biomaterial(s) can be provided, in which during its storage, adhesion of a minute amount of biomarker(s) contained in the biomaterial(s) to the surface of the container for storage can be suppressed, sample loss is reduced and it enables analysis with accuracy and highly precision.

### BRIEF DESCRIPTION OF THE DRAWINGS

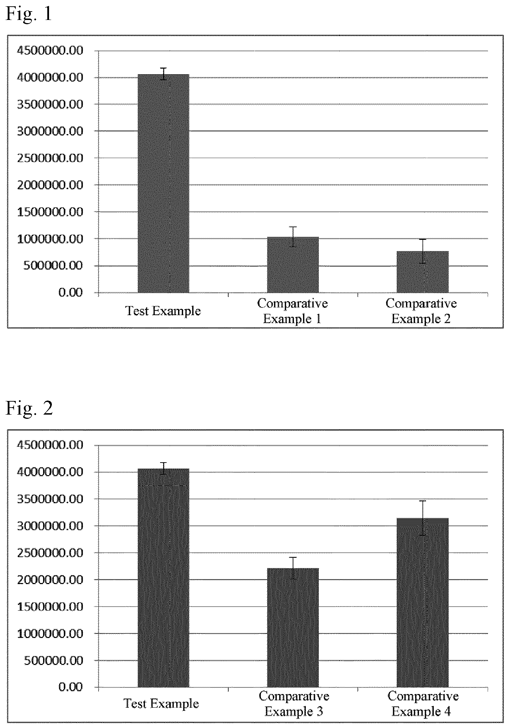

Fig 1 is, in Test Example 1, a graph showing the results of comparing the sum of the area of mass chromatogram of specific eight kinds of peptides in a sample using as a container for cryopreservation of a biomaterial, a container for pretreatment for HPLC measurement and a vial for HPLC measurement, the results of HPLC measurements of Test Example using a tube and vial made of PP which are coated in Example 1, Comparative Example 1 using an uncoated tube and vial made of PP, and Comparative Example 2 using a commercially available coating tube and vial (PROTEOSAVE (Registered Trademark)).
Fig. 2 is, in Test Example 1, a graph showing the results of comparing the sum of the area of mass chromatogram of specific eight kinds of peptides in a sample using as a container for cryopreservation of a biomaterial, a container for pretreatment for HPLC measurement and a vial for HPLC measurement, the results of HPLC measurements of Test Example using a tube and vial made of PP which are coated in Example 1, Comparative Example 3 using an uncoated tube and vial made of PP other than the container for cryopreservation of a biomaterial, and Comparative Example 4 using a commercially available coating tube and vial (PROTEOSAVE (Registered Trademark)) other than the container for cryopreservation of a biomaterial.

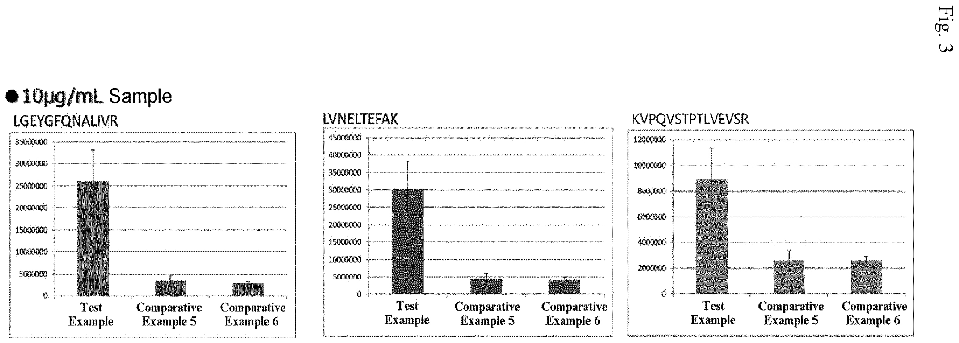

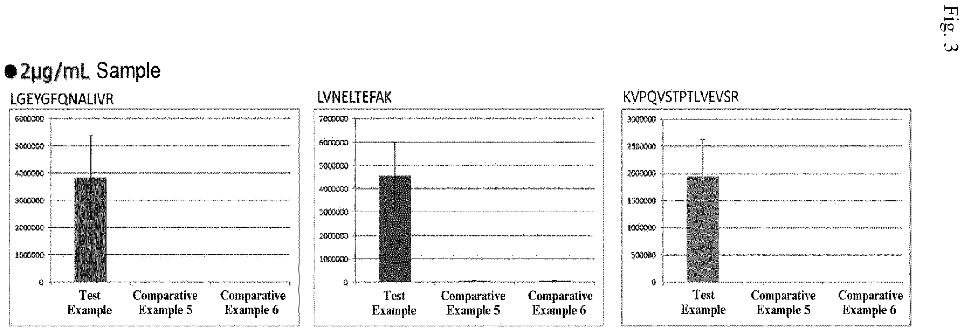

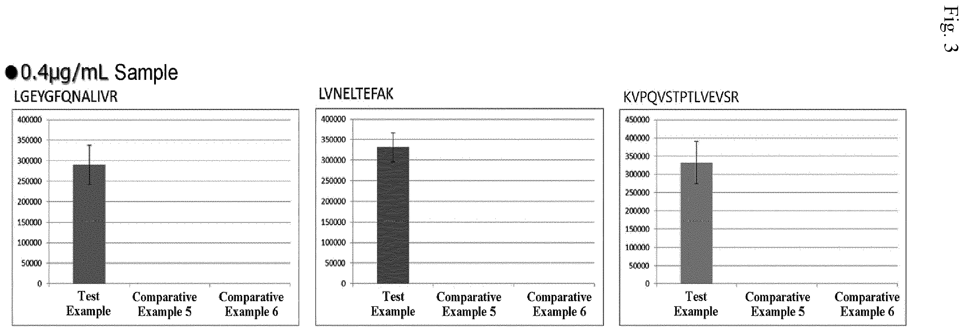

Fig. 3 is, in Test Example 2, a graph showing the results of comparing the sum of the area of mass chromatogram of specific three kinds of peptides in a sample using as a container for cryopreservation of a biomaterial with various kinds of concentrations, the results of HPLC measurements of Test Example using a tube and vial made of PP which are coated in Example 1, Comparative Example 5 using an uncoated tube made of PP, and Comparative Example 6 using a commercially available coating tube (PROTEOSAVE (Registered Trademark)).

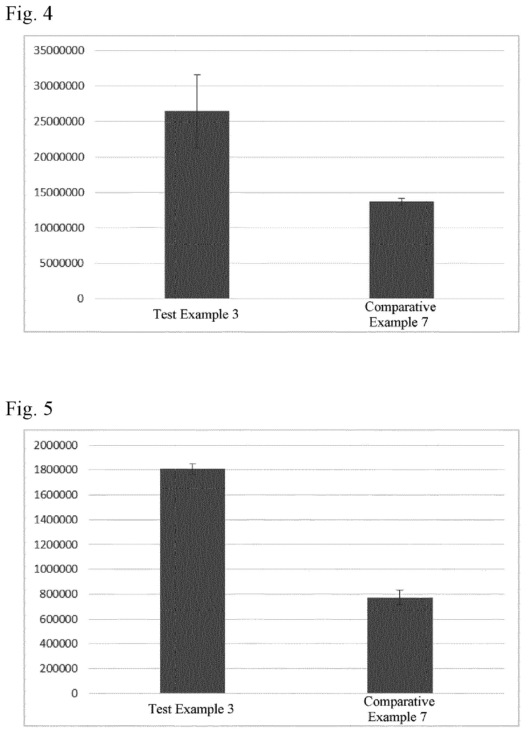

Fig. 4 is, in dilution operation (10-fold dilution 3 times) in Test Example 3, a graph comparing the area value of Aβ42 in a sample as a result of HPLC-mass spectrometry (SIM measurement) of Test Example 3 using a vial made of PP obtained in Example 2 as a dilution container and Comparative Example 7 using an uncoated vial made of PP.
Fig. 5 is, in dilution operation (10-fold dilution 5 times) in Test Example 3, a graph comparing the area value of Aβ42 in a sample as a result of HPLC-mass spectrometry (SIM measurement) of Test Example 3 using a vial made of PP obtained in Example 2 as a dilution container and Comparative Example 7 using an uncoated vial made of PP.

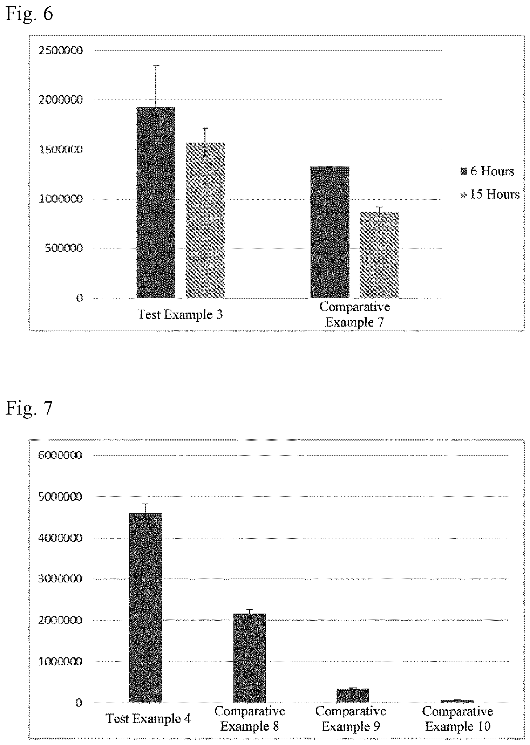

Fig. 6 is, in a further storage at 4°C of a sample after dilution operation (10-fold dilution 5 times) in Test Example 3, a graph comparing the area value of Aβ42 in a sample as a result of HPLC-mass spectrometry (SIM measurement) after 6 hours and after 15 hours of Test Example 3 using a vial made of PP obtained in Example 2 as a dilution container and Comparative Example 7 using an uncoated vial made of PP.
Fig. 7 is, in Test Example 4, a graph comparing the area value of Aβ42 in a sample as a result of HPLC-mass spectrometry (SIM measurement) of Test Example 4 using a tube and vial made of PP obtained in Example 2 as a dilution container and an HPLC measurement vial; Comparative Example 8 using a tube made of PP obtained in Example 2 as a dilution container and an uncoated vial made of PP as an HPLC measurement vial; Comparative Example 9 using an uncoated tube made of PP as a dilution container and a vial made of PP obtained in Example 2 as an HPLC measurement vial; and Comparative Example 10 using an uncoated tube and vial made of PP as a dilution container and an HPLC measurement vial.

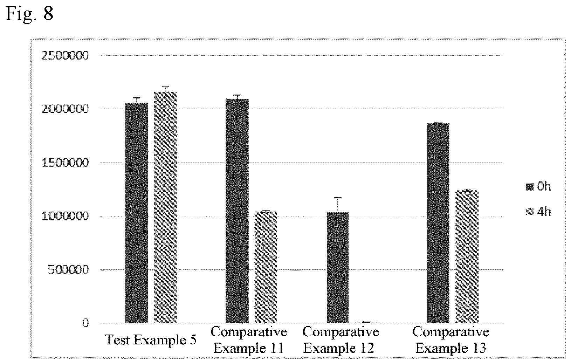

Fig. 8 is a graph comparing the area value of Aβ40 in the obtained sample as a result of HPLC-mass spectrometry (SIM measurement) of Test Example 5 using a tube and vial made of PP obtained in Example 2 as a dilution container and an HPLC measurement vial, with the area value of Aβ40 in the obtained sample as a result of HPLC-mass spectrometry (SIM measurement) of HPLC of Comparative Example 11 using a low adsorption vial made of PP manufactured by Shimadzu GLC Ltd.; Comparative Example 12 using an uncoated vial made of PP; and Comparative Example 13 using a vial made of PP manufactured by Waters K.K., as measurement vials.

### EMBODIMENTS TO CARRY OUT THE INVENTION

### «Explanation of the terms»

The terms used in the present invention have the following definitions, otherwise specifically mentioned.

In the present invention, the "halogen atom" means a fluorine atom, a chlorine atom, a bromine atom or an iodine atom.

In the present invention, the "alkyl group" means a linear or branched, saturated monovalent aliphatic hydrocarbon group. The "linear or branched alkyl group having 1 to 5 carbon atoms" may be mentioned, for example, a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group or a 1-ethylpropyl group. The "linear or branched alkyl group having 1 to 18 carbon atoms" may be mentioned, in addition to the examples of the "linear or branched alkyl group having 1 to 5 carbon atoms", a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group or an octadecyl group, or an isomer thereof. Similarly, the "linear or branched alkyl group having 1 to 10 carbon atoms" may be mentioned, in addition to the examples of the "linear or branched alkyl group having 1 to 5 carbon atoms", a hexyl group, a heptyl group, an octyl group, a nonyl group, a decyl group, or an isomer thereof.

In the present invention, the "linear or branched alkyl group having 1 to 5 carbon atoms which may be substituted by a halogen atom(s)" means either the above-mentioned linear or branched alkyl group having 1 to 5 carbon atoms, or the above-mentioned linear or branched alkyl group having 1 to 5 carbon atoms substituted by one or more of the above-mentioned halogen atoms. Examples of the "linear or branched alkyl group having 1 to 5 carbon atoms" are as mentioned above. On the other hand, the "linear or branched alkyl group having 1 to 5 carbon atoms substituted by one or more halogen atoms" means a group in which one or more optional hydrogen atoms of the above-mentioned linear or branched alkyl group having 1 to 5 carbon atoms is/are substituted by a halogen atom(s), and examples thereof may be mentioned a fluoromethyl group, a difluoromethyl group, a trifluoromethyl group, a chloromethyl group, a dichloromethyl group, a trichloromethyl group, a bromomethyl group, an iodomethyl group, a 2,2,2-trifluoroethyl group, a 2,2,2-trichloroethyl group, a perfluoroethyl group, a perfluorobutyl group or a perfluoropentyl group, etc.

In the present invention, the "ester bond" means -C(=O)-O- or -O-C(=O)-, the "amide bond" means -NHC(=O)- or -C(=O)NH- and the "ether bond" means -O-.

In the present invention, the "linear or branched alkylene group having 1 to 10 carbon atoms which may be substituted by a halogen atom(s)" means a linear or branched alkylene group having 1 to 10 carbon atoms or a linear or branched alkylene group having 1 to 10 carbon atoms substituted by one or more halogen atoms. Here, the "alkylene group" means a divalent organic group corresponding to the above-mentioned alkyl group. Examples of the "linear or branched alkylene group having 1 to 10 carbon atoms" may be mentioned a methylene group, an ethylene group, a propylene group, a trimethylene group, a tetramethylene group, a 1-methylpropylene group, a 2-methylpropylene group, a dimethylethylene group, an ethylethylene group, a pentamethylene group, a 1-methyl-tetramethylene group, a 2-methyl-tetramethylene group, a 1,1-dimethyl-trimethylene group, a 1,2-dimethyl-trimethylene group, a 2,2-dimethyl-trimethylene group, a 1-ethyl-trimethylene group, a hexamethylene group, an octamethylene group and a decamethylene group, etc., among these, an ethylene group, a propylene group, an octamethylene group and a decamethylene group are preferred, and, for example, a linear or branched alkylene group having 1 to 5 carbon atoms such as an ethylene group, a propylene group, a trimethylene group, a tetramethylene group, etc., are more preferred, and, in particular, an ethylene group or a propylene group is preferred. The "linear or branched alkylene group having 1 to 10 carbon atoms substituted by one or more halogen atoms" means a group in which one or more optional hydrogen atoms of the above-mentioned alkylene group is/are substituted by a halogen atom(s), and, in particular, a part or whole of the hydrogen atom(s) of the ethylene group or the propylene group is/are substituted by a halogen atom(s) is/are preferred.

In the present invention, the "alicyclic hydrocarbon group having 3 to 10 carbon atoms" means a monocyclic or polycyclic, saturated or partially unsaturated, monovalent aliphatic hydrocarbon group having 3 to 10 carbon atoms. Among these, a monocyclic or bicyclic, saturated monovalent aliphatic hydrocarbon group having 3 to 10 carbon atoms is preferred, and there may be mentioned, for example, a cycloalkyl group having 3 to 10 carbon atoms such as a cyclopropyl group, a cyclobutyl group and a cyclohexyl group, etc., or a bicycloalkyl group having 4 to 10 carbon atoms such as a bicyclo[3.2. 1]octyl group, a bornyl group and an isobornyl group, etc.

In the present invention, the "aryl group having 6 to 10 carbon atoms" means a monocyclic or polycyclic, monovalent aromatic hydrocarbon group having 6 to 10 carbon atoms, and there may be mentioned, for example, a phenyl group, a naphthyl group or an anthryl group, etc. The "aryl group having 6 to 10 carbon atoms" may be substituted by one or more of the above-mentioned "linear or branched alkyl group having 1 to 5 carbon atoms which may be substituted by a halogen atom(s)."

In the present invention, the "aralkyl group having 7 to 15 carbon atoms" means a group -R-R' (here, R represents the above-mentioned "linear or branched alkylene group having 1 to 5 carbon atoms", and R' represents the above-mentioned "aryl group having 6 to 10 carbon atoms"), and there may be mentioned, for example, a benzyl group, a phenethyl group or an α-methylbenzyl group, etc. The aryl portion of the "aralkyl group having 7 to 15 carbon atoms" may be substituted by one or more of the above-mentioned "linear or branched alkyl group having 1 to 5 carbon atoms which may be substituted by a halogen atom(s)."

In the present invention, the "aryloxyalkyl group having 7 to 15 carbon atoms" means a group -R-O-R' (here, R represents the above-mentioned "linear or branched alkylene group having 1 to 5 carbon atoms", and R' represents the above-mentioned "aryl group having 6 to 10 carbon atoms"), and there may be mentioned, for example, a phenoxymethyl group, a phenoxyethyl group or a phenoxypropyl group, etc. The aryl portion of the "aryloxyalkyl group having 7 to 15 carbon atoms" may be substituted by one or more of the above-mentioned "linear or branched alkyl group having 1 to 5 carbon atoms which may be substituted by a halogen atom(s)."

In the present invention, the "halide ion" means a fluoride ion, a chloride ion, a bromide ion or an iodide ion.

In the present invention, the "inorganic acid ion" means a carbonate ion, a sulfate ion, a phosphate ion, a hydrogen phosphate ion, a dihydrogen phosphate ion, a nitrate ion, a perchlorate ion or a borate ion.

As the above-mentioned An⁻, preferred are a halide ion, a sulfate ion, a phosphate ion, a hydroxide ion and an isothiocyanate ion, and particularly preferred is a halide ion.

In the present invention, the (meth)acrylate compound means both of an acrylate compound and a methacrylate compound. For example, the (meth)acrylic acid means acrylic acid and methacrylic acid.

In addition, in the present invention, the "anion" or the "anionic group" means a negative ion or a negative ionic group, and also contains a group capable of becoming a negative ion or a negative ionic group by dissociating in water. Similarly, in the present invention, the "cation" or the "cationic group" means a positive ion or a positive ionic group, and also contains a group capable of becoming a positive ion or a positive ionic group by dissociating in water.

### «Explanation of the present invention»

The container for storing a biomaterial of the present invention is provided with a coating which suppresses adhesion of a minute amount of a biomarker(s) contained in the biomaterial and reduces sample loss.

### <Biological sample>

The biomaterial in the present invention may be mentioned urine, blood (whole blood, serum, plasma, etc.), tissue (normal tissue, tumor tissue, pathological tissue, etc.), cerebrospinal fluid, synovial fluid, etc., collected from human, animal or plant, and is not particularly limited as long as it contains biological substances such as peptides, proteins, endogenous metabolites or genes, etc., that can be biomarkers.

### <Biomarker>

The biomarker in the present invention is a biological substance such as peptides, proteins, endogenous metabolites or genes, etc., contained in a biomaterial in a normal or pathological state, and means a material which becomes an index by correlating with a change in a disease or a response to a treatment. For example, there may be mentioned antigens such as tumor markers, pathogenic peptides or proteins such as amyloid and prions, antibodies and the like.

As the peptide biomarker and the protein biomarker, there may be mentioned, as specific examples, Aβ16, Aβ28, Aβ38, Aβ40, Aβ42, Aβ43, APP, α-Synuclein, a2-Macroglobulin, Acrp-30, Ang-1, Ang-2, Apo A-1, Apo B-100, AR, BAFF, BCA-1 (CXCL13), b-NGF, BDNF, BD-2, BMP-9, Cathepsin-D, CA 19-9, CA-125, Cathepsin S, CD-14, CD40L, CEA, c-MET, Clusterin, CNTF, COX-2, C-peptide, CRP, Eotaxin/ CXCL11, E-Cadherin, EGF, EGFR, ENA-78, Endoglin, Eotaxin, Eotaxin 3, ER (Epiregulin), ErbB2 (Her2), E-Selectin, FasL, FGF basic, Fibrinogen, Fibronectin, G-CSF, GDNF, GFAP, GM-CSF, gp130 (IL-6ST), GROa, GROg, HB-EGF, HE4/ WFDC2, HGF, HGH, HIV p24, I-309, ICAM-1, IFNα, IFNγ, IGFBP-1, IGFBP-2, IGFBP-3, IgE, IL-1a, IL-1b, IL-1ra, IL-2, IL-2Ra, IL-2Rg, IL-3, IL-4, IL-5, IL-6, IL-6R, IL-7, IL-8, IL-10, IL-12p40, IL-12p70, IL-13, IL-15, IL-17A, IL-17E, IL-18, IL-22 (Total), IL-23, IL-28A, IL-33, IL-36β, Insulin, IP-10, ITAC, KGF, Leptin, LIF, L-Selectin, Lymphotactin, MCP-1, MCP-2, MCP-3, MCP-4, M-CSF, MDC, MIF, MIG, MIP-1a, MIP-1b, MIP-3a, MIP-3b, MIP-4 (PARC), MMP-1, MMP-2, MMP-3, MMP-7, MMP-8, MMP-9, MMP-10, MMP-13, MPO, NAP-2, pNF-heavy, NF-light (Registered Trademark), NGAL, NSE, NT3, NT-proBNP, OPG, OPN, PAI-1 Active, PAI-1 Total, PAPP-A, PD-1, PD-L1, PDGF-AA, PDGF-AB, PDGF-BB, PEDF, PIGF, PLGF, Prolactin, PSA, P-Selectin, RAGE, RANK L, RANTES, RBP4, Resistin, SAA, SCF, SDF-1, TARC, Tau, P-Tau 231, TDP-43, TFF-3, TGFα, TGFβ, Tie-2, TIM-1, TIMP-1, TIMP-2, TNFα, TNFβ, TNF-RI, TNF-RII, TRAIL, Troponin-I, TSLP, TSP-1, TSP-2, TWEAK, UCH-L1, VCAM-1, VEGF-A, VEGF-C, VEGF-D, VEGF-R1, VEGF-R2 and VEGF-R3.

In the present invention, "reducing sample loss of a biomarker(s) contained in a biomaterial" means that, in HPLC measurement carried out by the method described in Examples (for example, Test Example 1) described later, a ratio (%) of sample loss ((sum of area of coated container)/(sum of area of uncoating container) × 100) when the sum of the area of the mass chromatogram of a predetermined component contained in a biomaterial stored in a container for storage provided with a coating according to the present invention and the sum of the area of the mass chromatogram of a predetermined component contained in the biomaterial stored in an uncoated container for storage as shown in Comparative Example 1 are compared to each other is 50% or less, preferably 30% or less, and further preferably 20% or less.

### <Coating>

The polymer contained in the coating provided with at least a part of a surface of the container for storing a biomaterial of the present invention is a copolymer containing a recurring unit which contains a group represented by the following formula (a) and a recurring unit which contains a group represented by the following formula (b): 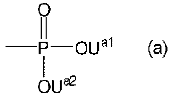 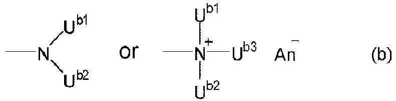 [(wherein U^{a1}, U^{a2}, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion].

In addition, the copolymer may further contain a recurring unit which contains a group represented by the following formula (c):

-R^{c} (c)

[wherein, R^{c} represents a linear or branched alkyl group having 1 to 18 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (here, the aryl portion may be substituted by a linear or branched alkyl group having 1 to 5 carbon atoms which may be substituted by a halogen atom(s))].

The copolymer according to the container for storing a biomaterial of the present invention is not particularly limited as long as it is a copolymer containing a recurring unit which contains the group represented by the above-mentioned formula (a), a recurring unit which contains the group represented by the above-mentioned formula (b), and optionally a recurring unit which contains the group represented by the above-mentioned formula (c). Incidentally, in the present invention, the recurring unit which contains the group represented by the above-mentioned formula (c) is different from the recurring unit which contains the group represented by the above-mentioned formula (a) and the recurring unit which contains the group represented by the above-mentioned formula (b). The copolymer is desirably a material obtained by radical polymerization of a monomer which contains a group represented by the above-mentioned formula (a), a monomer which contains a group represented by the above-mentioned formula (b), and optionally, a monomer which contains a group represented by the above-mentioned formula (c), but a material obtained by subjecting to polycondensation or polyaddition reaction may be also used. Examples of the copolymer may be mentioned a vinyl polymerized polymer in which olefins are reacted, polyamide, polyester, polycarbonate, polyurethane, etc., and among these, a vinyl polymerized polymer in which olefins are reacted or a (meth)acrylic polymer in which (meth)acrylate compounds are polymerized is desirable.

A ratio of the recurring unit which contains a group represented by the formula (a) in the copolymer is 3 mol% to 80 mol%. Incidentally, the copolymer may contain two or more kinds of the recurring units which contain a group represented by the formula (a).

A ratio of the recurring unit which contains a group represented by the formula (b) in the copolymer is 3 mol% to 80 mol%. Incidentally, the copolymer may contain two or more kinds of the recurring units which contain a group represented by the formula (b).

A ratio of the recurring unit which contains a group represented by the formula (c) in the copolymer may be the remainder subtracting the ratio of the above-mentioned formulae (a) and (b) from the whole copolymer and is, for example, 0 mol% to 90 mol%. Incidentally, the copolymer may contain two or more kinds of the recurring units which contain a group represented by the formula (c).

A preferred embodiment of the copolymer relating to the container for storing a biomaterial of the present invention is a copolymer containing the recurring units represented by the following formulae (a1) and (b1).

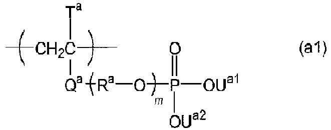

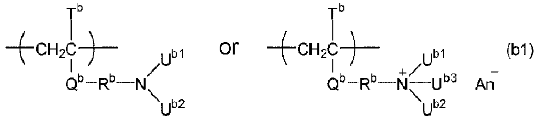

In the formula, T^{a} and T^{b} each independently represent a hydrogen atom or a linear or branched alkyl group having 1 to 5 carbon atoms, Q^{a} and Q^{b} each independently represent a single bond, an ester bond or an amide bond, R^{a} and R^{b} each independently represents a linear or branched alkylene group having 1 to 10 carbon atoms which may be substituted by a halogen atom(s), U^{a1}, U^{a2}, U^{b1}, U^{b2} and U^{b3} each independently represent a hydrogen atom or a linear or branched alkyl group having 1 to 5 carbon atoms, An⁻ represents an anion selected from the group consisting of a halide ion, an inorganic acid ion, a hydroxide ion and an isothiocyanate ion, and m represents an integer of 0 to 6.

The copolymer may further contain a recurring unit of the following formula (c1).

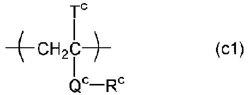

In the formula, T^{c} represents a hydrogen atom or a linear or branched alkyl group having 1 to 5 carbon atoms, Q^{c} represents a single bond, an ether bond or an ester bond, R^{c} represents a linear or branched alkyl group having 1 to 18 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (here, the above-mentioned aryl portion may be substituted by a linear or branched alkyl group having 1 to 5 carbon atoms which may be substituted by a halogen atom(s)).

In the formula (a1), m represents an integer of 0 to 6, preferably represents an integer of 1 to 6, more preferably represents an integer of 1 to 5, and particularly preferably 1.

A ratio of the recurring unit represented by the formula (a1) contained in the copolymer is 3 mol% to 80 mol%. Incidentally, the copolymer may contain two or more kinds of the recurring units represented by the formula (a1).

A ratio of the recurring unit represented by the formula (b1) contained in the copolymer is 3 mol% to 80 mol%. Incidentally, the copolymer may contain two or more kinds of the recurring units represented by the formula (b1).

A ratio of the recurring unit represented by the formula (c1) contained in the copolymer may be the remainder subtracting the above-mentioned formula (a1) and the formula (b1) from the whole copolymer, and it is, for example, 0 mol% to 90 mol%. Incidentally, the copolymer may contain two or more kinds of the recurring units represented by the formula (c1).

A preferred another embodiment of the copolymer relating to the container for storing a biomaterial of the present invention is a copolymer which is obtained by reacting (polymerizing) a monomer mixture containing compounds represented by the following formulae (A) and (B) : 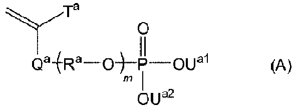 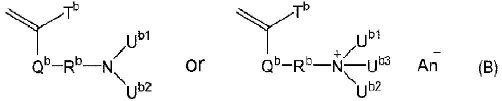 [here,
T^{a} and T^{b} each independently represent a hydrogen atom or a linear or branched alkyl group having 1 to 5 carbon atoms;
Q^{a} and Q^{b} each independently represent a single bond, an ester bond or an amide bond;
R^{a} and R^{b} each independently represent a linear or branched alkylene group having 1 to 10 carbon atoms which may be substituted by a halogen atom(s);
U^{a1}, U^{a2}, U^{b1}, U^{b2} and U^{b3} each independently represent a hydrogen atom or a linear or branched alkyl group having 1 to 5 carbon atoms;
An⁻ represents an anion selected from the group consisting of a halide ion, an inorganic acid ion, a hydroxide ion and an isothiocyanate ion; and
m represents an integer of 0 to 6]
in a solvent.

The copolymer may be a copolymer obtained from a monomer mixture which further contains a compound represented by the following formula (C): 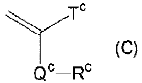 [here,
T^{c} represents a hydrogen atom or a linear or branched alkyl group having 1 to 5 carbon atoms;
Q^{c} represents a single bond, an ether bond or an ester bond;
R^{c} represents a linear or branched alkyl group having 1 to 18 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (here, the above-mentioned aryl portion may be substituted by a linear or branched alkyl group having 1 to 5 carbon atoms which may be substituted by a halogen atom(s))].

T^{a}, T^{b} and T^{c} are preferably a hydrogen atom, a methyl group or an ethyl group, and more preferably a hydrogen atom or a methyl group. Q^{a}, Q^{b} and Q^{c} are preferably a single bond or an ester bond, and more preferably an ester bond. R^{a} and R^{b} are preferably a linear or branched alkylene group having 1 to 5 carbon atoms, and more preferably a methylene group, an ethylene group or a propylene group. R^{c} is preferably a linear or branched alkyl group having 4 to 18 carbon atoms or a cycloalkyl group having 3 to 10 carbon atoms, and more preferably a butyl group, a pentyl group, a hexyl group or an isomer thereof, or a cyclohexyl group. U^{a1}, U^{a2}, U^{b1}, U^{b2} and U^{b3} are preferably a hydrogen atom, a methyl group, an ethyl group or a t-butyl group, U^{a1} and U^{a2} of the formula (a) is more preferably a hydrogen atom, and U^{b1}, U^{b2} and U^{b3} of the formula (b) is more preferably a hydrogen atom, a methyl group, an ethyl group or a t-butyl group.

Specific examples of the above-mentioned formula (A) may be mentioned vinylphosphonic acid, acid phosphoxyethyl (meth)acrylate, 3-chloro-2-acid phosphoxypropyl (meth)acrylate, acid phosphoxypropyl (meth)acrylate, acid phosphoxymethyl (meth)acrylate, acid phosphoxypolyoxyethylene glycol mono(meth)acrylate, acid phosphoxypolyoxypropylene glycol mono(meth)acrylate, etc., among these, vinylphosphonic acid, acid phosphoxyethyl methacrylate (=2-(methacryloyloxy)ethyl phosphate) or acid phosphoxypolyoxyethylene glycol monomethacrylate are preferably used, and most preferably acid phosphoxyethyl methacrylate (=2-(methacryloyloxy)-ethyl phosphate).

The structural formulae of vinylphosphonic acid, acid phosphoxyethyl methacrylate (=2-(methacryloyloxy)ethyl phosphate), acid phosphoxypolyoxyethylene glycol monomethacrylate and acid phosphoxypolyoxypropylene glycol monomethacrylate are represented by the following formula (A-1) to the formula (A-4), respectively.

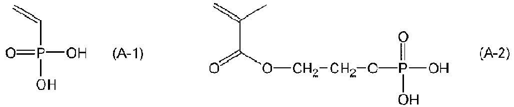

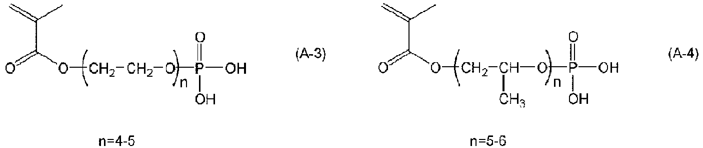

These compounds may contain a (meth)acrylate compound having two functional groups as represented by the general formula (D) or (E) mentioned later at the time of synthesis in some cases.

Specific examples of the above-mentioned formula (B) may be mentioned dimethylaminoethyl (meth)acrylate, diethylaminoethyl (meth)acrylate, dimethylaminopropyl (meth)acrylate, 2-(t-butylamino)ethyl (meth)acrylate, methacryloylcholine chloride, etc., among these, dimethylaminoethyl (meth)acrylate, methacryloylcholine chloride or 2-(t-butylamino)ethyl (meth)acrylate is preferably used, and dimethylaminoethyl (meth)acrylate is most preferably used.

The structural formulae of dimethylaminoethyl acrylate (=acrylic acid 2-(dimethylamino)ethyl), diethylaminoethyl methacrylate (=methacrylic acid 2-(diethyl-amino)ethyl), dimethylaminoethyl methacrylate (=methacrylic acid 2-(dimethylamino)-ethyl), methacryloylcholine chloride and 2-(t-butylamino)ethyl methacrylate (=methacrylic acid 2-(t-butylamino)ethyl) are represented by the following formula (B-1) to the formula (B-5), respectively.

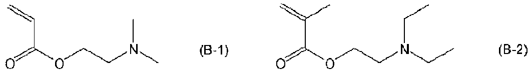

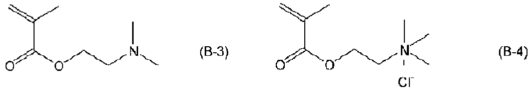

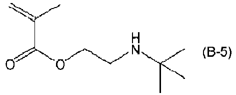

Specific examples of the above-mentioned formula (C) may be mentioned linear or branched alkyl esters of (meth)acrylic acid such as butyl (meth)acrylate, 2-ethylhexyl (meth)acrylate, lauryl (meth)acrylate, stearyl (meth)acrylate, etc.; cyclic alkyl esters of (meth)acrylic acid such as cyclohexyl (meth)acrylate, isobornyl (meth)acrylate, etc.; aralkyl esters of (meth)acrylic acid such as benzyl (meth)acrylate, phenethyl (meth)acrylate, etc.; styrene-based monomers such as styrene, methylstyrene, chloromethylstyrene, etc.; vinyl ether-based monomers such as methyl vinyl ether, butyl vinyl ether, etc.; and vinyl ester-based monomers such as vinyl acetate, vinyl propionate, etc. Among these, butyl (meth)acrylate or cyclohexyl (meth)acrylate is preferably used.

The structural formulae of butyl methacrylate (=methacrylic acid butyl) and cyclohexyl methacrylate (=methacrylic acid cyclohexyl) are represented by the following formula (C-1) and the formula (C-2), respectively.

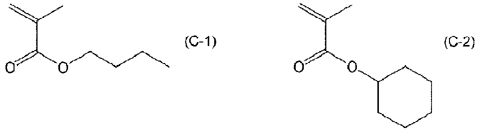

In another embodiment of the copolymer according to the present invention, in addition to the compounds represented by the above-mentioned formulae (A), (B) and, if necessary, (C), an optional fourth component may be further copolymerized. For example, as the fourth component, a (meth)acrylate compound having two or more functional groups may be copolymerized, and a part of the polymer may be partially three-dimensionally crosslinked. Such a fourth component may be mentioned, for example, a bifunctional monomer represented by the following formula (D) or (E): 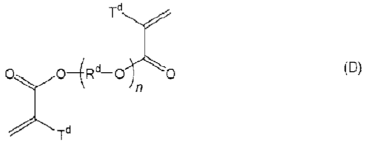 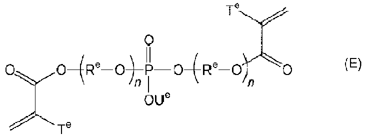 [here, T^{d}, T^{e} and U^{e} each independently represent a hydrogen atom or a linear or branched alkyl group having 1 to 5 carbon atoms, R^{d} and R^{e} each independently represent a linear or branched alkylene group having 1 to 10 carbon atoms which may be substituted by a halogen atom(s); and n represents an integer of 1 to 6]. That is, the copolymer relating to the present invention preferably contains a crosslinking structure derived from such a bifunctional monomer.

In the formulae (D) and (E), T^{d} and T^{e} are preferably each independently a hydrogen atom, a methyl group or an ethyl group, and more preferably each independently a hydrogen atom or a methyl group.

In the formula (E), U^{e} is preferably a hydrogen atom, a methyl group or an ethyl group, and more preferably a hydrogen atom.

In the formula (D), R^{d} is preferably a linear or branched alkylene group having 1 to 3 carbon atoms which may be substituted by a halogen atom(s), more preferably each independently an ethylene group or a propylene group, or an ethylene group or a propylene group which is substituted by one chlorine atom, and particularly preferably an ethylene group or a propylene group. Also, in the formula (D), n preferably represents an integer of 1 to 5, and particularly preferably 1.

In the formula (E), R^{e} is preferably a linear or branched alkylene group having 1 to 3 carbon atoms which may be substituted by a halogen atom(s), more preferably each independently an ethylene group or a propylene group, or an ethylene group or a propylene group which is substituted by one chlorine atom, and particularly preferably an ethylene group or a propylene group. Also, in the formula (E), n preferably represents an integer of 1 to 5, and particularly preferably 1.

The bifunctional monomer represented by the formula (D) may be preferably mentioned ethylene glycol di(meth)acrylate, triethylene glycol di(meth)acrylate and propylene glycol di(meth)acrylate.

The bifunctional monomer represented by the formula (E) may be preferably mentioned bis(methacryloyloxymethyl) phosphate, bis[(2-methacryloyloxy)ethyl] phosphate, bis[3-(methacryloyloxy)propyl] phosphate, or a bifunctional monomer derived from the above-mentioned formula (A-3) or (A-4).

Among the formula (D) and the formula (E), the bifunctional monomer represented by the formula (E) is more preferably used.

In addition, as the trifunctional (meth)acrylate compound, there may be mentioned phosphinylidynetris(oxy-2,1-ethanediyl) triacrylate.

Among these fourth components, in particular, ethylene glycol dimethacrylate, among the bi-functional monomers derived from the above-mentioned formulae (A-3) and (A-4), a dimethacrylate having a recurring unit of ethylene glycol and propylene glycol, bis[2-(methacryloyloxy)ethyl] phosphate, and among the bi-functional monomer derived from the above-mentioned formulae (A-3) and (A-4), a dimethacrylate having a recurring unit of ethylene glycol and propylene glycol through a phosphate ester group are preferable, and the structural formulae thereof are represented by the following formulae (D-1) to (D-3) and formulae (E-1) to (E-3), respectively.

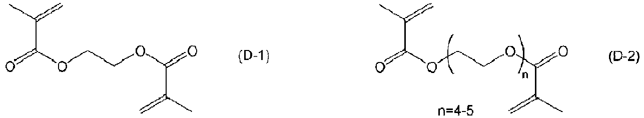

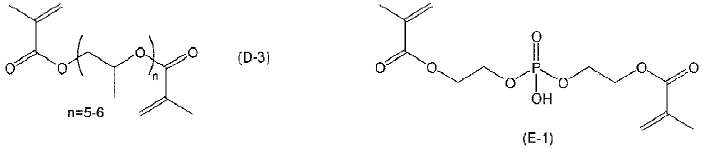

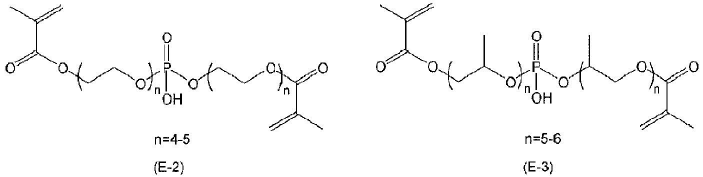

Among these, the formulae (E-1) to (E-3) are particularly preferable.

In the copolymer, one or two or more kinds of these fourth components may be contained.

A ratio of the fourth component in the above-mentioned copolymer, for example, a crosslinking structure derived from the bi-functional monomer represented by the above-mentioned formula (D) or (E) is 0 mol% to 50 mol%, preferably5 mol% to 45 mol%, and most preferably 10 mol% to 40 mol%.

A ratio of the compound represented by the formula (A) in the whole monomer constituting the above-mentioned copolymer is 3 mol% to 80 mol%. In addition, the compound represented by the formula (A) may be two or more kinds.

A ratio of the compound represented by the formula (B) in the whole monomer constituting the above-mentioned copolymer is 3 mol% to 80 mol%. In addition, the compound represented by the formula (B) may be two or more kinds.

A ratio of the compound represented by the formula (C) in the whole monomer constituting the above-mentioned copolymer may be the remainder subtracting the ratio of the above-mentioned formulae (A) and (B) therefrom and is, for example, 0 mol% to 90 mol%. In addition, the compound represented by the formula (C) may be two or more kinds.

In the copolymer according to the present invention, an ethylenically unsaturated monomer, or a polysaccharide or derivatives thereof may be further copolymerized as a further optional fifth component. Examples of the ethylenically unsaturated monomer may be mentioned one or two or more kinds of the ethylenically unsaturated monomers selected from the group consisting of a (meth)acrylic acid and an ester thereof; vinyl acetate; vinylpyrrolidone; ethylene; vinyl alcohol; and a hydrophilic functional derivative thereof. Examples of the polysaccharides or derivatives thereof may be mentioned a cellulose-based polymer such as hydroxyalkyl cellulose (for example, hydroxyethyl cellulose or hydroxypropyl cellulose), etc., starch, dextran and curdlan.

Examples of the hydrophilic functional groups of the hydrophilic functional derivatives may be mentioned phosphoric acid, phosphonic acid and an ester structure thereof; a betaine structure; an amide structure; an alkylene glycol residue; an amino group; and a sulfinyl group, etc.

Here, the phosphoric acid and an ester structure thereof mean a group represented by the following formula: 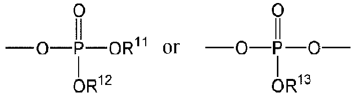 [here, R¹¹, R¹² and R¹³ each independently represent a hydrogen atom or an organic group (for example, a linear or branched alkyl group having 1 to 5 carbon atoms, etc.)], the phosphonic acid and an ester structure thereof mean a group represented by the following formula: 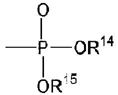 [here, R¹⁴ and R¹⁵ each independently represent a hydrogen atom or an organic group (for example, a linear or branched alkyl group having 1 to 5 carbon atoms, etc.)]. Examples of the ethylenically unsaturated monomer having such a structure may be mentioned acid phosphoxyethyl (meth)acrylate, vinyl phosphonic acid, etc.

The betaine structure means a monovalent or a divalent group of a compound having an amphoteric center of a quaternary ammonium type cation structure and an acidic anion structure and may be mentioned, for example, a phosphorylcholine group: 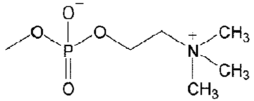 Examples of the ethylenically unsaturated monomer having such a structure may be mentioned 2-methacryloyloxyethylphosphorylcholine (MPC), etc.

The amide structure means a group represented by the following formula: 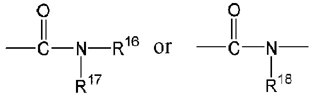 [here, R¹⁶, R¹⁷ and R¹⁸ each independently represent a hydrogen atom or an organic group (for example, a methyl group, a hydroxymethyl group or a hydroxyethyl group, etc.)].
Examples of the ethylenically unsaturated monomer having such a structure may be mentioned (meth)acrylamide, N-(hydroxymethyl) (meth)acrylamide, etc. Further, the monomer or the polymer having such a structure is disclosed in, for example, JP 2010-169604A, etc.

The alkylene glycol residue means an alkyleneoxy group (-Alk-O-) which remains after a hydroxyl group(s) at one side terminal or both terminals of the alkylene glycol (HO-Alk-OH; here, Alk is a linear or branched alkylene group having 1 to 10 carbon atoms) is/are subjected to condensation reaction with other compound(s), and a poly(alkyleneoxy) group in which alkyleneoxy units are repeated is also included. Examples of the ethylenically unsaturated monomer having such a structure may be mentioned 2-hydroxyethyl (meth)acrylate, methoxypolyethylene glycol (meth)acrylate, etc. Further, the monomer or the polymer having such a structure is disclosed in, for example, JP 2008-533489A, etc.

The amino group means a group represented by the formula: -NH₂, -NHR¹⁹ or -NR²⁰R²¹ [here, R¹⁹, R²⁰ and R²¹ each independently represent an organic group (for example, a linear or branched alkyl group having 1 to 5 carbon atoms, etc.)]. In the amino group of the present invention, a quaternalized or chlorinated amino group is included. Examples of the ethylenically unsaturated monomer having such a structure may be mentioned dimethylaminoethyl (meth)acrylate, 2-(t-butylamino)ethyl (meth)acrylate, methacryloylcholine chloride, etc.

The sulfinyl group means a group represented by the following formula: 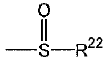 [here, R²² is an organic group (for example, an organic group having 1 to 10 carbon atoms, preferably an alkyl group having 1 to 10 carbon atoms which has one or more hydroxyl groups, etc.)].
As the polymer having such a structure, there may be mentioned a copolymer disclosed in the publication of JP 2014-48278A, etc.

### <Producing method of copolymer>

The copolymer according to the present invention can be synthesized by the methods of radical polymerization, anion polymerization, cation polymerization, etc., which are general synthetic methods of an acrylic polymer or a methacrylic polymer, etc. The form can be taken various kinds of methods such as solution polymerization, suspension polymerization, emulsion polymerization, bulk polymerization, etc.

As the solvent for the polymerization reaction, it may be water, phosphate-buffered saline or an alcohol such as ethanol, etc., or a mixed solvent in which these are combined, and desirably contains water or ethanol. Further, it is preferable to contain 10% by mass or more and 100% by mass or less of water or ethanol. Moreover, it is preferable to contain 50% by mass or more and 100% by mass or less of water or ethanol. Furthermore, it is preferable to contain 80% by mass or more and 100% by mass or less of water or ethanol. Still further, it is preferable to contain 90% by mass or more and 100% by mass or less of water or ethanol. It is preferable that the sum of water and ethanol is 100% by mass.

As a reaction concentration, for example, a concentration of the compound represented by the above-mentioned formula (A) or the formula (B) in the reaction solvent is preferably made 0.01% by mass to 4% by mass. If the concentration exceeds 4% by mass, for example, the copolymer sometimes gelled in the reaction solvent due to strong association property possessed by the phosphate group represented by the formula (A). If the concentration is less than 0.01% by mass, a concentration of the obtained varnish is too low, so that it is difficult to prepare a coating agent for obtaining a coating film with a sufficient film thickness. The concentration is more preferably 0.01% by mass to 3% by mass, for example, 3% by mass, 2% by mass or 1% by mass.

Also, in the synthesis of the copolymer according to the present invention, for example, after preparing a salt described in the following formula (1), in some cases, polymerization may be carried out with the compound represented by the formula (C) to prepare a copolymer.

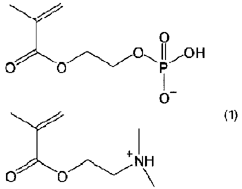

The phosphate group-containing monomer is a monomer easily associated, so that it may be added dropwise in the reaction solvent little by little so as to rapidly disperse therein when it is added dropwise into the reaction system.

Further, the reaction solvent may be heated (for example, 40°C to 100°C) so as to raise solubility of the monomer and the polymer.

In order to efficiently proceed the polymerization reaction, it is desirable to use a polymerization initiator. Examples of the polymerization initiator, 2,2'-azobis(isobutyronitrile), 2,2'-azobis(2-methylbutyronitrile), 2,2'-azobis(2,4-dimethylvaleronitrile) (available from Wako Pure Chemical Industries, Ltd.; V-065, 10 hour half-life temperature; 51°C), 4,4'-azobis(4-cyanovaleric acid), 2,2'-azobis(4-methoxy-2,4-dimethylvaleronitrile), 1,1'-azobis(cyclohexane-1-carbonitrile), 1-[(1-cyano-1-methylethyl)azo]formamide, 2,2'-azobis[2-(2-imidazolin-2-yl)propane], 2,2'-azobis[2-(2-imidazolin-2-yl)propane], 2,2'-azobis(2-methylpropionamidine) dihydrochoride, 2,2'-azobis[2-methyl-N-(2-hydroxyethyl)propionamide] (available from Wako Pure Chemical Industries, Ltd.; VA-086, 10 hour half-life temperature; 86°C), benzoyl peroxide (BPO), 2,2'-azobis[N-(2-carboxyethyl)-2-methylpropionamidine] n-hydrate (available from Wako Pure Chemical Industries, Ltd.; VA-057, 10 hour half-life temperature; 57°C), 4,4'-azobis(4-cyanopentanoic acid) (available from Wako Pure Chemical Industries, Ltd.; V-501), 2,2'-azobis[2-(2-imidazolin-2-yl)propane] disulfate dihydrate (available from Wako Pure Chemical Industries, Ltd.; VA-046B, 10 hour half-life temperature; 46°C), 2,2'-azobis[2-(2-imidazolin-2-yl)propane] (available from Wako Pure Chemical Industries, Ltd.; VA-061, 10 hour half-life temperature; 61°C), 2,2'-azobis(2-amidinopropane) dihydrochloride (available from Wako Pure Chemical Industries, Ltd.; V-50, 10 hour half-life temperature; 56°C), peroxydisulfuric acid or t-butylhydroperoxide, etc., may be used.

When solubility in water, ion balance and interaction with the monomer are taking into consideration, it is preferably selected from 2,2'-azobis[2-methyl-N-(2-hydroxyethyl)propionamide], 2,2'-azobis[N-(2-carboxyethyl)-2-methylpropionamidine] n-hydrate, 4,4'-azobis(4-cyanopentanoic acid), 2,2'-azobis[2-(2-imidazolin-2-yl)-propane] dihydrochloride, 2,2'-azobis[2-(2-imidazolin-2-yl)propane] disulfate dihydrate, 2,2'-azobis[2-(2-imidazolin-2-yl)propane], 2,2'-azobis(2-amidinopropane) dihydrochloride and peroxydisulfuric acid.

When solubility in an organic solvent, ion balance and interaction with the monomer are taking into consideration, it is desirable to use 2,2'-azobis(2,4-dimethylvaleronitrile) or 2,2'-azobis(isobutyronitrile).

As an amount of the polymerization initiator to be added, it is 0.05% by mass to 10% by mass based on the total weight of the monomer to be used for polymerization.

The reaction conditions are to carry out stirring in a reaction vessel by heating to 50°C to 200°C with an oil bath, etc., for 1 hour to 48 hours, more preferably at 80°C to 150°C for 5 hours to 30 hours to proceed the polymerization reaction whereby a copolymer according to the present invention can be obtained. The reaction atmosphere is preferably a nitrogen atmosphere.

As a reaction procedure, whole reaction substances may be charged in the reaction solvent at room temperature and then these may be heated to the above-mentioned temperature and polymerized, or all or a part of the mixture of the reaction substances may be added dropwise little by little into a previously heated solvent.

According to the latter reaction procedure, a varnish containing the copolymer of the present invention can be prepared by the producing method including the steps of adding a mixture containing the compounds of the above-mentioned formulae (A), (B) and optionally (C), the solvent and the polymerization initiator dropwise into a solvent maintained at a temperature higher than the 10 hour half-life temperature of the polymerization initiator, and reacting (polymerizing) the same.

The molecular weight of the copolymer according to the present invention may be about several thousands to several millions, and preferably 5,000 to 5,000,000. It is further preferably 10,000 to 2,000,000, and most preferably 5,000 to 1,000,000. In addition, it may be either of a random copolymer, a block copolymer or a graft copolymer, there is no particular limitation on the copolymerization reaction itself for manufacturing the copolymer, and a conventionally known method synthesizing in a liquid can be used such as polymerization utilizing radical polymerization, ionic polymerization, photopolymerization or emulsion polymerization. These can be used any of the copolymers of the present invention solely or a plurality of the copolymers are mixed and the ratio thereof may be changed depending on the objective uses.

The coating agent to be used for forming a coating onto the container for storing a biomaterial according to the present invention may be prepared by diluting a desired copolymer with a desired solvent to a predetermined concentration, depending on the cases.

Such a solvent may be mentioned water, phosphate buffered saline (PBS) and an alcohol. The alcohol may be mentioned an alcohol having 2 to 6 carbon atoms, for example, ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (=neopentyl alcohol), 2-methyl-1-propanol, 2-methyl-1-butanol, 2-methyl-2-butanol (=t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol and cyclohexanol, which may be used alone or a mixed solvent of a combination thereof, and from the viewpoint of dissolution of the copolymer, it is preferably selected from water, PBS, ethanol, propanol and a mixed solvent thereof, and more preferably selected from water, ethanol and a mixed solvent thereof.

Further, the coating agent may be prepared from a varnish containing the copolymer. The varnish containing the copolymer can be prepared by, for example, the producing method containing the step of reacting (polymerizing) the compounds represented by the above-mentioned formulae (A), (B) and optionally (C) in a solvent with a total concentration of the compounds of 0.01% by mass to 20% by mass.

A concentration of the solid content in the coating agent is desirably 0.01 to 50% by mass to uniformly form a coating film. Also, a concentration of the copolymer in the coating agent is preferably 0.01 to 4% by mass, more preferably 0.01 to 3% by mass, particularly preferably 0.01 to 2% by mass, and further preferably 0.01 to 1% by mass. If the concentration of the copolymer is less than 0.01% by mass, a coating film having a sufficient film thickness cannot be formed since the concentration of the copolymer in the coating agent is too low, while if it exceeds 4% by mass, storage stability of the coating agent becomes worse, and there is a possibility to cause precipitation or gellation of the dissolved material.

Further, in addition to the above-mentioned copolymer and the solvent, other substances may be added to the coating agent within the range that does not impair the properties of the obtainable coating film, if necessary. As the other substances, there may be mentioned preservatives, surfactants, primers that enhance adhesive property with the substrate, fungicides and sugars, etc.

In order to adjust ion balance of the copolymer in the coating agent, a step of adjusting a pH in the coating agent in advance may be contained. Adjustment of the pH may be carried out, for example, by adding a pH adjusting agent to the composition containing the above-mentioned copolymer and the solvent and made the pH of the composition 2 or more, preferably 2 to 9, more preferably 2.5 to 8.5, and further preferably 3 to 8. A kind of the usable pH adjusting agent and an amount thereof can be optionally selected depending on the concentration of the above-mentioned copolymer or an existing ratio of the anion and the cation thereof, and the like.

Examples of the pH adjusting agent may be mentioned organic amines such as ammonia, diethanolamine, pyridine, N-methyl-D-glucamine, tris(hydroxymethyl)-aminomethane, etc.; alkali metal hydroxides such as potassium hydroxide, sodium hydroxide, etc.; alkali metal halides such as potassium chloride, sodium chloride, etc.; inorganic acids such as sulfuric acid, phosphoric acid, hydrochloric acid, carbonic acid, etc., or alkali metal salts thereof; quaternary ammonium cations such as choline, etc.; or a mixture thereof (for example, a buffer such as phosphate buffered saline, etc.). Among these, ammonia, diethanolamine, sodium hydroxide, choline, N-methyl-D-glucamine and tris(hydroxymethyl)aminomethane are preferable, and in particular, ammonia, diethanolamine, sodium hydroxide and choline are preferable.

The container for storing a biomaterial of the present invention has a coating formed by the above-mentioned coating agent at least a part of the surface of the container for storing. Specifically, it has the coating at least a part of the surface at the inside and/or outside of the container, which can be contacted with the biomaterial containing a biomarker.

### <Container>

The container to which the copolymer is coated, which constitutes the container for storing a biomaterial of the present invention, may be containers with an optional shape capable of using in this field of the art, and may be mentioned, for example, dishes or schale (petri dish) generally used for storing the biomaterial such as petri dishes, dishes for tissue culture, multi dishes, etc., flasks such as a cell culture flask, a spinner flask, etc., bags such as plastic bags, Teflon (Registered Trademark) bags, culture bags, etc., vials such as screw vials, etc., tubes such as culture tubes, centrifugal tubes, microtubes, etc., trays, roller bottles and the like. It is preferably mentioned vials and tubes.

Also, the material of the container may be used glass, a metal, a metal containing compound or a semi-metal containing compound, activated charcoal or a resin. The metal may be mentioned a typical metal: (an alkali metal: Li, Na, K, Rb and Cs; an alkaline earth metal: Ca, Sr, Ba and Ra), a magnesium group element: Be, Mg, Zn, Cd and Hg; an aluminum group element: Al, Ga and In; a rare earth element: Y, La, Ce, Pr, Nd, Sm and Eu; a tin group element: Ti, Zr, Sn, Hf and Pb, Th; an iron group element: Fe, Co and Ni; a vanadium group element: V, Nb and Ta, a chromium group element: Cr, Mo, W and U; a manganese group element: Mn and Re; a noble metal: Cu, Ag and Au; and a platinum group element: Ru, Rh, Pd, Os, Ir and Pt, etc. The metal containing compound or the semi-metal containing compound may be mentioned, for example, ceramics comprising a metal oxide as a basic component, which are a sintered body baked by a heat treatment at a high temperature, a semiconductor such as silicon, an inorganic solid material including a molded product of an inorganic compound such as a metal oxide or a semi-metal oxide (silicon oxide, alumina, etc.), a metal carbide or a semi-metal carbide, a metal nitride or a semi-metal nitride (silicon nitride, etc.), a metal boride or a semi-metal boride, etc., aluminum, nickel-titanium and stainless (SUS304, SUS316, SUS316L, etc.).

As the resin, it may be either of a natural resin or a derivative thereof, or a synthetic resin, as the natural resin, there may be preferably used cellulose, cellulose triacetate (CTA), nitrocellulose (NC), cellulose to which dextran sulfate has been fixed, etc., and as the synthetic resin, there may be preferably used polyacrylonitrile (PAN), polyester-based polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester, polypropylene (PP), polyvinylidene fluoride (PVDF), polyether sulfone (PES), polycarbonate (PC), polyvinyl chloride (PVC), polytetrafluoroethylene (PTFE), ultra-high molecular weight polyethylene (UHPE), polydimethylsiloxane (PDMS), acrylonitrile-butadiene-styrene resin (ABS) or Teflon (Registered Trademark). In the manufacture of the container for storing a biomaterial of the present invention, at the time of coating the copolymer to exist at least a part of the surface of the container, it is not necessary to treat it at a high temperature, so that a resin having low heat resistance, etc., can be also applied.

A material(s) of the container may be one kind or a combination of two or more kinds. Among these materials, it is preferably glass, silicon, silicon oxide, polystyrene (PS), polypropylene (PP), polyether sulfone (PES), polyethylene terephthalate (PET), polycarbonate (PC), polyvinyl chloride (PVC), Teflon (Registered Trademark), cycloolefin polymer (COP), polydimethylsiloxane (PDMS) or stainless (SUS304, SUS316, SUS316L, etc.) alone, or a combination selected from these, and particularly preferably glass, polystyrene (PS), polypropylene (PP) or stainless (SUS304, SUS316, SUS316L, etc.).

### <Manufacturing method of container for storing biomaterial>

The present invention relates to a method for manufacturing a container for storing a biomaterial having a coating onto at least a part of the surface of the container, which comprises a step of contacting the coating agent as mentioned above with at least a part of the surface of the container. There is no particular limitation on the contact of the coating agent and the surface of the container, and there may be used a method in which the container is dipped in the coating agent, the coating agent is added to the container and allowed to stand for a predetermined time, or the coating agent is coated onto the surface of the container, etc., and a method in which the coating agent is added to the container and allowed to stand for a predetermined time is preferable. Addition can be carried out, for example, by adding the coating agent with 0.5 to 1-fold amount of the whole volume of the container using a syringe, etc. Standing is carried out by appropriately selecting a time and a temperature depending on the material of the container and the kind of the coating agent and carried out, for example, for 5 minutes to 24 hours, preferably for 30 minutes to 3 hours, at 10 to 35°C, preferably at 20 to 30°C, and most preferably at 25°C. According to the procedure, a container for storing a biomaterial having a coating onto at least a part of the surface of the container, preferably whole surface thereof can be manufactured.

Also, the coating on the surface of the container obtained by such a method can be used as a container for storing a biomaterial after the step of contacting with at least a part of the above-mentioned surface of the container, preferably after the step of adding a coating agent and allowing to stand for a predetermined time, and when an excess coating agent is remained, it is removed from the container and without passing through the drying step as such, or after the step of washing using water or a medium (for example, water, buffer, medium, etc.) of the material to be applied to storage.

That is, after the step of contacting at least a part of the above-mentioned surface of the container, preferably after the step of adding a coating agent and allowing to stand for a predetermined time, within 48 hours, preferably within 24 hours, further preferably within 12 hours, further preferably within 6 hours, further preferably within 3 hours, and further preferably within 1 hour, when an excess coating agent is remained, it is removed from the container and without passing through the drying step as such, or after washing using water or a medium (for example, water, buffer, medium, etc., particularly preferably medium (for example, DMEM medium (Dulbecco's modified Eagle medium)) of a sample to be applied to storage, it can be used as a container for storing a biomaterial.

The container may be applied to a drying step. The drying step is carried out under atmosphere or under vacuum, preferably at a temperature in the range of -200°C to lower than 200°C. By the drying step, the solvent in the above-mentioned coating agent is removed, and also the formula (a) and the formula (b) of the copolymer relating to the present invention are formed an ion bonding with each other to completely adhere to the substrate.

The coating can be formed, for example, by the drying at room temperature (10°C to 35°C, preferably at 20°C to 30°C, for example, 25°C), and it may be dried, for example, at 40°C to 50°C to form the coating more rapidly. In addition, a drying step by the freeze-drying method at an extremely low temperature to a low temperature (-200°C to around -30°C) may be used. The freeze drying is called as vacuum freeze-drying, and is a method in which a material to be desired to dry is cooled with a refrigerant in general and remove the solvent under a vacuum state by sublimation. A general refrigerant to be used in the freeze drying may be mentioned a mixed medium of dry ice and methanol (-78°C), liquid nitrogen (-196°C), etc.

If the drying temperature is lower than -200°C, an unusual refrigerant must be used which lacks versatility, and a long time is required for solvent sublimation so that efficiency is poor. If the drying temperature is 200°C or higher, ionic bonding reaction on the surface of the coating proceeds excessively, whereby the surface thereof loses hydrophilicity and adhesion inhibiting ability of the biomarker is not exhibited. More preferred drying temperature is 10°C to 180°C, and more preferred drying temperature is 25°C to 150°C.

The coating of the present application is produced through the simple and easy step as mentioned above.

In addition, in order to remove impurities, unreacted monomer, etc., remained in the coating, and further, to adjust ion balance of the copolymer in the coating, it may be carried out a step of washing with at least one kind of the solvent selected from water and an aqueous solution containing an electrolyte. Washing is desirably running water washing or ultrasonic wave washing, etc. The above-mentioned water and the aqueous solution containing an electrolyte may be a material heated, for example, in the range of 40°C to 95°C. The aqueous solution containing an electrolyte is preferably PBS, physiological saline (containing only sodium chloride), Dulbecco's phosphate-buffered saline, Tris buffered physiological saline, HEPES buffered physiological saline and veronal buffered physiological saline, and particularly preferably PBS. After adhesion, the coating film does not dissolve even when it is washed with water, PBS and alcohol, etc., and still remains firmly adhered to the substrate. Even if cells or proteins are adhered to the formed coating, these can be easily removed by washing with water, etc., thereafter, so that the surface of the container onto which the coating of the present invention has been formed has an ability to inhibit adhesion of the biomarkers.

A film thickness of the coating applied onto the surface of the container of the present invention can be appropriately adjusted according to the shape of the container or the kind of the sample, etc., and may be substantially uniform over the whole surface of the container or may be partially ununiform, which is not particularly limited, and preferably 10 to 1000Å, further preferably 10 to 500Å, and most preferably 10 to 300Å.

### <Other embodiments>

The present invention also relates to use of a container for storing a peptide which is provided with a coating on a surface of the container, which contains a copolymer containing a recurring unit which contains a group represented by the formula (a) and a recurring unit which contains a group represented by the formula (b), and optionally a recurring unit which contains a group represented by the formula (c), at least a part of the surface of the container. The meanings of the copolymer, coating and container, etc., in such a container for storage are as described above. Also, the peptide is preferably a material contained in a biomaterial to be applied to mass analysis.

The present invention also relates to use of a container for fragmentation treatment of a protein and/or a peptide, which is provided with a coating on a surface of the container, which contains a copolymer containing a recurring unit which contains a group represented by the formula (a) and a recurring unit which contains a group represented by the formula (b), and optionally a recurring unit which contains a group represented by the formula (c), at least a part of the surface of the container. The meanings of the copolymer, coating and container, etc., in such a treatment container are as described above. The fragmentation treatment means treating a protein or a peptide with a proteolytic enzyme (protease), etc., such as trypsin, etc., to decompose it into fragment (=a peptide) having a smaller molecular weight. A sample in which a protein and/or a peptide sample is/are fragmented with protease which contains resulting many peptides can be applied to mass analysis.

The present invention also relates to a method which is an analytical method of a fragmented protein and/or peptide, which comprises preparing a sample containing a protein and/or a peptide which is/are an analytical object(s), subjecting the protein and/or the peptide in the sample to fragmentation treatment in a container provided with a coating containing a copolymer which contains a recurring unit which contains a group represented by the following formula (a) and a recurring unit which contains a group represented by the following formula (b), and optionally a recurring unit which contains a group represented by the formula (c) at least a part of a surface of the container, and applying the fragmented protein and/or peptide in the sample to a measurement apparatus. The meanings of the copolymer, coating and container, etc., in such a method are as described above. In the analytical method of a fragmented protein and/or peptide of the present invention, by subjecting a protein and/or a peptide which is/are an analytical object(s) to fragmentation treatment in a container having a specific coating, adhesion of the fragmented peptide to the surface of the treatment container is suppressed to reduce sample loss, and an analysis which is accurate and has high accuracy is possible so that the matching rate of the sequence is improved.

The present invention is also a method which is a method for measuring an absolute amount(s) of a biomarker(s) in a solution, which comprises a step of preparing a sample containing a biomarker(s) which is/are an analytical object(s), a step of containing and/or storing a solution containing a biomarker(s) contained in a biomaterial in a container for storing a biomaterial having a coating which contains a copolymer containing a recurring unit which contains a group represented by the following formula (a) and a recurring unit which contains a group represented by the following formula (b), and optionally a recurring unit which contains a group represented by the formula (c) on at least a part of a surface of the container, and then, a step of measuring an absolute amount(s) of the biomarker(s). The meanings of the copolymer, coating and container, etc., in such a method are as described above.

When the above-mentioned sample is subjected to pretreatment such as dilution before measurement, it is preferable that a container for the pretreatment also has a coating containing the above-mentioned copolymer on at least a part of the surface. When the dilution treatment is carried out a plurality of times, it is preferable that all the containers for the pretreatment are provided with a coating containing the above-mentioned copolymer on at least a part of the surface.

According to the above-mentioned method, the deficient amount of the biomarker(s) in the above-mentioned sample containing the biomarker(s) can be suppressed to a low level, and it is possible to measure the absolute amount of the biomarker(s) in the sample accurately. When a plurality of biomarkers is present in the above-mentioned sample, it is possible to measure the contained ratio of each accurately by measuring the respective absolute amounts.

Described is a method for reducing loss of a biomarker(s) contained in a biomaterial, which comprises preparing the biomaterial containing the biomarker(s) which is/are an analytical object(s), and containing and/or storing a solution containing the biomaterial in a container for storing the biomaterial having a coating which contains a copolymer containing a recurring unit which contains a group represented by the following formula (a) and a recurring unit which contains a group represented by the following formula (b), and optionally a recurring unit which contains a group represented by the formula (c) on at least a part of a surface of the container. The meanings of the biomaterial, copolymer, coating and container, etc., in such a method are as described above.

The present invention also relates to use of a coating for reducing loss of a biomarker(s) contained in a biomaterial, which contains a copolymer containing a recurring unit which contains a group represented by the formula (a) and a recurring unit which contains a group represented by the formula (b), and optionally a recurring unit which contains a group represented by the formula (c) in a container for containing and/or storing a solution containing the biomaterial. In such a use, it has a coating containing the copolymer at least a part of a surface of the container, and preferably at least a part or all of the surface inside and/or outside of the container that can be come into contact with a biomaterial.

### EXAMPLES

Hereinafter, the present invention will be explained in more detail based on Synthetic Example, Examples, Test Example, etc., but the present invention is not limited to these.

### <Synthetic Example 1>

To 390 g of ethanol was added 260 g of acid phosphoxyethyl methacrylate (product name; Phosmer M, available from UniChemical Co, non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and 310 g of choline (48-50% aqueous solution: available from Tokyo Chemical Industry Co., Ltd.) was added thereto while cooling the mixture to 35°C or lower and the mixture was stirred until it became uniform. To the mixture were added 220 g of 80 % aqueous methacryloylcholine chloride solution (available from Tokyo Chemical Industry Co., Ltd.) and 300 g of butyl methacrylate (available from Tokyo Chemical Industry Co., Ltd.), and 260 g of ethanol was additionally added and the mixture was stirred.

Further, an aqueous solution in which 22 g of 2,2'-azobis(N-(2-carboxyethyl)-2-methylpropionamidine) n-hydrate (product name; VA-057, available from Wako Pure Chemical Industries, Ltd.) had been dissolved in 230 g of pure water was added to the above-mentioned solution while maintaining the mixture to 35°C or lower, and a mixed solution in which all the above-mentioned materials were contained which became uniform by sufficiently stirring was introduced into a three-necked flask through a dropping pump. On the other hand, 650 g of pure water and 980 g of ethanol were added to a three-necked flask equipped with a cooling tube separately and subjected to nitrogen flow, and heated to a reflux temperature while stirring. While maintaining the state, the above-mentioned mixed liquid was added dropwise into a boiled liquid of pure water and ethanol by a dropping pump through a Teflon tube over 1.5 hours. After the dropping, the mixture was stirred under heating for 2 hours while maintaining the above-mentioned circumstance. By cooling after 2 hours, 3,610 g of a varnish containing a copolymer having a solid content of about 24.20% were obtained. A weight average molecular weight of the obtained liquid by GFC was about 23,225.

### <Preparation Example 1>

To 568 g of the varnish containing the copolymer obtained in the above-mentioned Synthetic Example 1 were added 157 g of 1 mol/L hydrochloric acid (1N) (available from KANTO CHEMICAL CO., INC.), 1,697 g of pure water and 4,456 g of ethanol and the mixture was sufficiently stirred to prepare a coating agent. The pH was 2.6.

### <Example 1>

The coating agent obtained in Preparation Example 1 was charged each 1.0 mL in a j acket tube made of polypropylene (PP) (manufactured by FCR & Bio CO., LTD., #JRDS-0M), each 1.5 mL in a microtube made of PP, and each 300 µL in a screw vial made of PP (manufactured by YMC CO., LTD., #XRAV1103-P), and allowed to stand at 25°C for 0.5 hour. After removing the coating agent, each was dried at 25°C for 3 hours. Thereafter, these were sufficiently washed with pure water to obtain tubes and vials made of PP in which a coating film had been formed.

### <Test Example 1>

Immunoglobulin G (IgG, Sigma-Aldrich, #I4506) was dissolved in phosphate buffered saline (PBS) to prepare a solution having a concentration of 2 µg/mL, and 1 mL thereof was dispensed in the jacket tube made of PP obtained in Example 1 in which a coating film had been formed. As Comparative Example, an IgG solution having the same concentration was dispensed in an uncoated jacket tube made of PP or a PROTEOSAVE (trademark registration) SS 1.5 mL slim tube (manufactured by Sumitomo Bakelite Co., Ltd., #MS-4202X) (each is made Comparative Example 1 and Comparative Example 2). The sample after dispensation was stored under frozen (-80°C) for 5 days.

The samples after the storage were allowed to stand at room temperature for 30 minutes, then stirred by vortex, and 200 µL thereof was transferred to a tube for pretreatment (the microtube made of PP to which the coating film has been formed obtained in Example 1). In addition, with regard to Comparative Example 1 and Comparative Example 2, each was transferred to an uncoated microtube made of PP or a PROTEOSAVE (trademark registration) SS 1.5 mL microtube (manufactured by Sumitomo Bakelite Co., Ltd., #MS-4215M). Further, as Comparative Example 3 and Comparative Example 4, the samples stored in the tube to which the coating has been applied were each transferred to an uncoated microtube made of PP or a PROTEOSAVE (trademark registration) SS 1.5 mL microtube (manufactured by Sumitomo Bakelite Co., Ltd., #MS-4215M).

**[Table 1]**

| | Storage container | Pretreated container | HPLC vial |
|---|---|---|---|
| Test Example | Said coating | Said coating | Said coating |
| Comparative Example 1 | Uncoating | Uncoating | Uncoating |
| Comparative Example 2 | PROTEOSAVE | PROTEOSAVE | PROTEOSAVE |
| Comparative Example 3 | Said coating | Uncoating | Uncoating |
| Comparative Example 4 | Said coating | PROTEOSAVE | PROTEOSAVE |

With regard to the above-mentioned samples, 800 µL of cold acetone (available from FUJIFILM Wako Pure Chemical Corporation) was added to each of them, and the protein was allowed to stand at -20°C for 1 hour or longer. Thereafter, the protein was precipitated by centrifugation at 4°C, 16,000 × g for 10 minutes and after removing the supernatant, and the protein was dissolved again by adding 100 µL of Tris-hydrochloric acid buffer (pH 8.3). To the sample were added 2.1 µL of 0.5 mol/L dithiothreitol (DTT, available from Thermo Fisher Scientific K.K.) and 11.5 µL of 0.5 mol/L iodoacetamide (available from FUJIFILM Wako Pure Chemical Corporation), and after stirring well, it was allowed to stand in a dark place for 20 minutes. Thereafter, 460 µL of cold acetone (available from FUJIFILM Wako Pure Chemical Corporation) was added thereto, and after stirring well, it was allowed to stand at -20°C for 1 hour. After stirring well, centrifugation was carried out at 4°C, 16,000 × g for 5 minutes and the supernatant was removed. Subsequently, 50 µL of 90% cold acetone was added thereto, and after stirring well, it was again centrifuged at 4°C, 16,000 × g for 5 minutes and the supernatant was removed. Further, 100 µL of 50 mmol/L ammonium hydrogen carbonate solution (pH 8.0) (available from FUJIFILM Wako Pure Chemical Corporation) was added thereto, and the protein pellets were dissolved again. To the solution was added 2 µL of 0.1 mg/mL trypsin (available from Sigma-Aldrich) dissolved in 50 mmol/L acetic acid solution, and the mixture was shaken at 37°C overnight. Thereafter, 10 µL of 20% trifluoroacetic acid (available from FUJIFILM Wako Pure Chemical Corporation) was added thereto to prepare a sample, and the sample was transferred to an HPLC vial (the screw vial made of PP to which the coating film has been formed obtained in Example 1). With regard to Comparative Examples, each was transferred to an uncoated screw vial made of PP or an extremely low adsorption 0.3 mL vial ProteoSave (manufactured by AMR Inc., #PSVial).

As described above, the sample transferred to the HPLC vial was measured by HPLC-mass spectrometry according to the following conditions.

### HPLC conditions

HPLC device: Nexera X2 (Shimadzu Corporation)
Mass analysis device: Orbitrap Fusion (Thermo Fisher Scientific K.K.)
Column: PLRP-S (300Å, 5 µm, 150 × 2.1 mm, manufactured by Agilent Technologies)
Eluent: 0.1% aqueous formic acid solution/0.1% formic acid acetonitrile
Gradient: 90/10 → 50/50 (0-10 min), 50/50 → 5/95 (10-12 min), 5/95 (12-17 min)
Flow rate: 0.25 mL/mL
Column temperature: 55°C
Injection amount: 5 µL

After the HPLC measurement, mass chromatogram of each of eight peptides (VVSVLTVLHQDWLNGK, TPEVTCVVVDVSHEDPEVK, FNWYVDGVEVHNAK, GPSVFPLAPSSK, DTLMISR, VVSVLTVVHQDWLNGK, GPSVFPLAPCSR, STSESTAALGCLVK) was extracted, the area was calculated, and the results that the sum thereof was compared with those of Comparative Example 1 and Comparative Example 2 are shown in Fig. 1. As a result, it was clarified that the area value was significantly larger when the coating was applied in all three steps as compared with Comparative Examples. Also, the results compared with those of Comparative Example 3 and Comparative Example 4 are shown in Fig. 2. As a result, it was clarified that the area value was significantly larger when the coating was applied only in the steps of the pretreatment container or the HPLC vial as compared with Comparative Examples.

### <Test Example 2>

Bovine serum albumin (BSA, Sigma-Aldrich, #A9647) was dissolved in phosphate buffered saline (PBS) to prepare solutions having a concentration of 0.4 µg/mL, 2 µg/mL and 10 µg/mL, and each 1 mL thereof was dispensed in the jacket tube made of PP to which the coating film has been formed obtained in Example 1. As Comparative Examples, an IgG solution having the same concentration was dispensed in an uncoated jacket tube made of PP or a PROTEOSAVE (trademark registration) SS 1.5 mL slim tube (manufactured by Sumitomo Bakelite Co., Ltd., #MS-4202X). The sample after dispensation was stored under frozen (-80°C) for 12 days (each is made Comparative Example 5 and Comparative Example 6).

The samples after the storage were allowed to stand at room temperature for 30 minutes, then stirred by vortex, and 200 µL thereof was transferred to a microtube made of PP. For the subsequent operations, the same operations were carried out for Comparative Examples.

With regard to the above-mentioned samples, 800 µL of cold acetone (available from FUJIFILM Wako Pure Chemical Corporation) was added to each of them, and the protein was allowed to stand at -20°C for 1 hour or longer. Thereafter, the protein was precipitated by centrifugation at 4°C, 16,000 × g for 10 minutes and after removing the supernatant, and the protein was dissolved again by adding 100 µL of Tris-hydrochloric acid buffer (pH 8.3). To the sample were added 2.1 µL of 0.5 mol/L dithiothreitol (DTT, available from Thermo Fisher Scientific K.K.) and 11.5 µL of 0.5 mol/L iodoacetamide (available from FUJIFILM Wako Pure Chemical Corporation), and after stirring well, it was allowed to stand in a dark place for 20 minutes. Thereafter, 460 µL of cold acetone (available from FUJIFILM Wako Pure Chemical Corporation) was added thereto, and after stirring well, it was allowed to stand at -20°C for 1 hour. After stirring well, centrifugation was carried out at 4°C, 16,000 × g for 5 minutes and the supernatant was removed. Subsequently, 50 µL of 90% cold acetone was added thereto, and after stirring well, it was again centrifuged at 4°C, 16,000 × g for 5 for 5 minutes and the supernatant was removed. Further, 100 µL of 50 mmol/L ammonium hydrogen carbonate solution (pH 8.0) (available from FUJIFILM Wako Pure Chemical Corporation) was added thereto, and the protein pellets were dissolved again. To the solution was added 2 µL of 0.1 mg/mL trypsin (available from Sigma-Aldrich) dissolved in 50 mmol/L acetic acid solution, and the mixture was shaken at 37°C overnight, and thereafter, 10 µL 20% trifluoroacetic acid (available from FUJIFILM Wako Pure Chemical Corporation) was added thereto to prepare a sample, and the solvent was removed with a centrifuge concentrator (EZ-2 Elite, manufactured by Genavac). To the residue was added 100 µL of 0.1% aqueous formic acid solution : acetonitrile mixed solution (50 : 50) to prepare a sample, and the sample was transferred to an HPLC vial (a screw vial made of PP).

As described above, the sample transferred to the HPLC vial was measured by HPLC-mass spectrometry according to the following conditions.

### HPLC conditions

HPLC device: Nexera X2 (Shimadzu Corporation)
Mass analysis device: Orbitrap Fusion (Thermo Fisher Scientific K.K.)
Column: PLRP-S (300Å, 5 µm, 150 × 2.1 mm, manufactured by Agilent Technologies)
Eluent: 0.1% aqueous formic acid solution/0.1% formic acid acetonitrile
Gradient: 90/10 → 50/50 (0-10 min), 50/50 → 5/95 (10-12 min), 5/95 (12-17 min)
Flow rate: 0.25 mL/mL
Column temperature: 55°C
Injection amount: 10 µL

After the HPLC measurement, mass chromatogram of each of three peptides (LGEYGFQNALIVR, LVNELTEFAK, KVPQVSTPTLVEVSR) was extracted, the area was calculated, and the results that the sum thereof was compared with those of Comparative Example 5 and Comparative Example 6 are shown in Fig. 3. As a result, it was clarified that the area value was significantly larger when the coating was applied in the step of storage as compared with Comparative Examples.

Also, in each measurement, in the amino acid sequence of BSA, the results of calculating the proportion of the identified peptide are shown in Table 2. It was clarified that the proportion dominantly identified was higher when the coating was carried out as compared with Comparative Examples, and was also superior in terms of qualitative analysis.

**[Table 2]**

| Example | Sequence Coverage |
|---|---|
| Test Example | 36. 7%±2. 3% |
| Comparative Example 5 | 28. 7%± 0.6% |
| Comparative Example 6 | 21. 0% ± 2. 6% |

### <Example 2>

Each 300 µL of the coating agents obtained in Preparation Example 1 was charged in a vial made of polypropylene (PP) (manufactured by Shimadzu GLC Ltd., #GLCTV-PP), and allowed to stand at 25°C for 0.5 hour. After removing the coating agent from the vial, it was dried at 25°C for 3 hours. Thereafter, the vial was thoroughly washed with pure water to obtain a vial made of PP on which a coating film was formed.

Each 1.5 mL of the coating agents obtained in Preparation Example 1 was charged in a 1.5 mL tube made of PP (manufactured by Thermo Fisher Scientific K.K. or Sarstedt K.K.), and allowed to stand at 25°C for 30 minutes. After removing the coating agent, it was dried at 25°C for 3 hours. Thereafter, the tube was thoroughly washed with pure water to obtain a 1.5 mL tube made of PP on which a coating film was formed.

### <Test Example 3>

Amyloid βa protein fragments 1-42 (Aβ42, PEPTIDE INSTITUTE, INC., #4349-v) were dissolved in a mixed solution of 0.3% aqueous ammonia and acetonitrile with 1 : 1 (volume ratio) to prepare a solution having a concentration of 1,000 ppm. Using the vial made of PP to which the coating film has been formed obtained in Example 2, 10-fold dilution was carried out 3 times or 5 times with a mixed solution of 0.3% aqueous ammonia and acetonitrile with 1 : 1 (volume ratio).

As Comparative Example, an Aβ42 dilute solution in which the same 10-fold dilution was carried out 3 times or 5 times was prepared using an uncoated vial made of PP (which is made Comparative Example 7).

As described above, the sample in the HPLC vial made of PP was measured by HPLC-mass spectrometry according to the following conditions.

### HPLC conditions

HPLC device: LC-20AD (Shimadzu Corporation)
Mass analysis device: LTQ-Orbitrap (Thermo Fisher Scientific K.K.)
Column: ACQUITY UPLC CSH C18 (2.1 × 150 mm, 1.7 µm, manufactured by Nihon Waters K.K.)
Eluent: 0.3% aqueous ammonia solution/acetonitrile
Gradient: 90/10 (0-1 min), 90/10 → 45/55 (1-6.5 min), 45/55 (6.5-6.7 min), 45/55 → 10/90 (6.7-7 min), 10/90 (7-9 min)
Flow rate: 0.20 mL/min
Column temperature: 55°C
Injection amount: 5 µL
Ionization method: ESI
Observation mode: Positive
tSIM (m/z): 1129.32
Scan width (m/z): 2

The area of tetravalent ions with m/z = 1129.32 was calculated by SIM measurement (selective ion measurement, Selective Ion Monitoring), and the results compared with Comparative Example 7 are shown in Table 3 (results of 10-fold dilution 3 times) and Table 4 (results of 10-fold dilution 5 times) (N = 2). Also, these are shown in Fig. 4 (results of 10-fold dilution 3 times) and Fig. 5 (results of 10-fold dilution 5 times). As a result, it was clarified that in the samples of both concentrations, the area value was significantly larger when the coating was performed as compared with Comparative Example.

**[Table 3]**

| | Area value | |
|---|---|---|
| | 1 | 2 |
| Test Example 3 | 21263450 | 31564925 |
| Comparative Example 7 | 13228320 | 14170202 |

**[Table 4]**

| | Area value | |
|---|---|---|
| | 1 | 2 |
| Test Example 3 | 1851177 | 1765259 |
| Comparative Example 7 | 713979 | 831917 |

In addition, a sample having a concentration of diluted 10-fold 5 times was prepared in the same procedure as mentioned above, stored at 4°C, and after 6 hours and 15 hours, an area of a tetravalent ion of m/z = 1129.32 was calculated by SIM measurement, and the results compared with Comparative Example 7 are shown in Table 5 and Fig. 6 (N = 2). As a result, it was clarified that the area value was significantly larger when the coating was carried out for both 6 hours and 15 hours as compared with Comparative Example. Further, the changed amount from 6 hours to 15 hours ((average area value of 6 hours - average area value of 15 hours) × average area value of 100/6 hours) was 18.7% in Test Example 3 and 34.3% in Comparative Example 7, so that it was found that the changed amount was significantly lower when the coating was carried out as compared with Comparative Example.

**[Table 5]**

| | Area value | | | |
|---|---|---|---|---|
| | 6 hours | | 15 hours | |
| | 1 | 2 | 1 | 2 |
| Test Example 3 | 2343844 | 1514270 | 1420963 | 1715982 |
| Comparative Example 7 | 1317983 | 1331879 | 818821 | 922223 |

### <Test Example 4>

Amyloid βa protein fragments 1-42 (Aβ42, PEPTIDE INSTITUTE, INC., #4349-v) were dissolved in a mixed solution of 0.3% aqueous ammonia and acetonitrile with 9 : 1 (volume ratio) to prepare a solution having a concentration of 1,000 ppm. Using a 1.5 mL tube made of PP to which the coating film has been formed obtained in Example 2, 10-fold dilution was carried out 4 times with a mixed solution of 0.3% aqueous ammonia and acetonitrile with 9 : 1 (volume ratio). The obtained solution was transferred to the vial made of PP to which the coating film has been formed obtained in Example 2, and measurement was carried out by HPLC-mass spectrometry according to the following conditions.

As Comparative Examples, the following three were carried out. In Comparative Example 8, an Aβ42-diluted solution in which the same 10-fold dilution 4 times was carried out was prepared using a 1.5 mL tube made of PP to which the coating film has been formed, and the obtained solution was transferred to an uncoated vial made of PP, and measurement was carried out by HPLC-mass spectrometry according to the following conditions. In Comparative Example 9, an Aβ42-diluted solution in which the same 10-fold dilution 4 times was carried out was prepared using an uncoated 1.5 mL tube made of PP, and the obtained solution was transferred to a vial made of PP to which the coating film has been formed obtained in Example 2, and measurement was carried out by HPLC-mass spectrometry according to the following conditions. In Comparative Example 10, an Aβ42-diluted solution in which the same 10-fold dilution 4 times was carried out was prepared using an uncoated 1.5 mL tube made of PP, and the obtained solution was transferred to an uncoated vial made of PP, and measurement was carried out by HPLC-mass spectrometry according to the following conditions.

**[Table 6]**

| | Dilution container | HPLC vial |
|---|---|---|
| Test Example 4 | Said coating | Said coating |
| Comparative Example 8 | Said coating | Uncoating |
| Comparative Example 9 | Uncoating | Said coating |
| Comparative Example 10 | Uncoating | Uncoating |

### HPLC conditions

HPLC device: LC-20AD (Shimadzu Corporation)
Mass analysis device: LTQ-Orbitrap (Thermo Fisher Scientific K.K.)
Column: ACQUITY UPLC CSH C18 (2.1 × 150 mm, 1.7 µm, manufactured by Nihon Waters K.K.)
Eluent: 0.3% aqueous ammonia solution/acetonitrile =80/20 (0-13 min)
Flow rate: 0.20 mL/min
Column temperature: 55°C
Injection amount: 5 µL
Ionization method: ESI
Observation mode: Positive
tSIM (m/z): 1129.32
Scan width (m/z): 2

The area of tetravalent ions with m/z = 1129.32 was calculated by SIM measurement (selective ion measurement, Selective Ion Monitoring), and the results comparing Test Example 4 and Comparative Examples 8, 9 and 10 are shown in Table 7 and Fig. 7. As a result, it was clarified that the area value was significantly larger when the coating was carried out to both the dilution container and the HPLC vial.

**[Table 7]**

| | 1 | 2 | AVERAGE |
|---|---|---|---|
| Test Example 4 | 4492030 | 4679156 | 4585593 |
| Comparative Example 8 | 2125487 | 2202581 | 2164034 |
| Comparative Example 9 | 342393 | 353574 | 347983.5 |
| Comparative Example 10 | 57647 | 84794 | 71220.5 |

### <Test Example 5>

Amyloid βa protein fragments 1-40 (Aβ40, PEPTIDE INSTITUTE, INC., #4307-v) were dissolved in a mixed solution of 0.3% aqueous ammonia and acetonitrile with 9 : 1 (volume ratio) to prepare a solution having a concentration of 1,000 ppm. Using a 1.5 mL tube made of PP to which the coating film has been formed obtained in Example 2, 10-fold dilution was carried out 4 times with a mixed solution of 0.3% aqueous ammonia and acetonitrile with 9 : 1 (volume ratio). The obtained solution was transferred to a vial made of PP to which the coating film has been formed obtained in Example 2, and measurement was carried out by HPLC-mass spectrometry according to the following conditions. The measurement was carried out twice, i.e., the measurement carried out immediately after transferring to the vial (0h) and the measurement carried out when 4 hours had passed in an autosampler maintained at 4°C (4h).

As Comparative Examples, the following three were carried out. In Comparative Example 11, a low adsorption vial made of PP of TORAST-H Bio Vial (manufactured by Shimadzu GLC Ltd., #GLCTV-H-BIO) was used as an analytical vial, and measurement was carried out under the same conditions as in Test Example 5. In Comparative Example 12, an uncoated vial made of PP was used as an analytical vial, and measurement was carried out under the same conditions as in Test Example 5. In Comparative Example 13, a low adsorption vial made of PP of QuanRecovery with MaxPeak HPS 300 µL vials (manufactured by Waters K.K., #186009186) was used as an analytical vial, and measurement was carried out under the same conditions as in Test Example 5.

### HPLC conditions

HPLC device: LC-20AD (Shimadzu Corporation)
Mass analysis device: LTQ-Orbitrap (Thermo Fisher Scientific K.K.)
Column: ACQUITY UPLC CSH C18 (2.1 × 150 mm, 1.7 µm, manufactured by Nihon Waters K.K.)
Eluent: 0.3% aqueous ammonia solution/acetonitrile = 80/20 (0-13 min)
Flow rate: 0.20 mL/min
Column temperature: 55°C
Injection amount: 5 µL
Ionization method: ESI
Observation mode: Positive
tSIM (m/z): 1,083.29
Scan width (m/z): 2

The area of tetravalent ions with m/z = 1083.29 was calculated by SIM measurement (selective ion measurement, Selective Ion Monitoring), and the results comparing Test Example 5 and Comparative Examples 11, 12 and 13 are shown in Table 8 and Fig. 8. As a result, it was clarified that the area value was significantly larger when the coating was carried out to the HPLC vial. Also, in Comparative Examples 11, 12 and 13, the area value was significantly reduced in the measurement after 4 hours, while in Test Example 5, the area value was maintained at the same level.

**[Table 8]**

| | 0h | | 4h | |
|---|---|---|---|---|
| | 1 | 2 | 1 | 2 |
| Test Example 5 | 2005759 | 2110274 | 2121014 | 2208675 |
| Comparative Example 11 | 2056350 | 2133280 | 1037243 | 1052935 |
| Comparative Example 12 | 901184 | 1174149 | 8934 | 11402 |
| Comparative Example 13 | 1863656 | 1866685 | 1234647 | 1251934 |

Accordingly, when analyzing an extremely minute amount of peptides known to be easily adsorbed, such as Aβ42 and Aβ40, it was confirmed that the coating suppresses adsorption, which reduces sample loss and is capable of carrying out analysis with accuracy and highly precision.

## Claims

1. Use of a container for storing a biomaterial for reducing sample loss of a biomarker(s) contained in the biomaterial, which comprises
a coating containing a copolymer which contains a recurring unit which contains a group represented by the following formula (a) and a recurring unit which contains a group represented by the following formula (b): 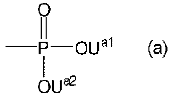 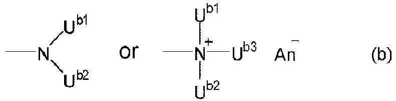
wherein
U^{a1}, U^{a2}, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion,
provided with at least a part of a surface of the container.

2. Use according to Claim 1, wherein the copolymer contains a recurring unit which contains a group represented by the following formula (c):
-R^{c} (c)
wherein
R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms where the aryl portion may be substituted by a linear or branched alkyl group having 1 to 5 carbon atoms which may be substituted by a halogen atom(s).

3. Use according to Claim 1 or 2, wherein the biomarker(s) is/are an antibody or amyloid.

4. Use of a container according to any one of claims 1 to 2 for storing a peptide.

5. Use of the container for storing a peptide according to Claim 4, wherein the peptide is contained in a biomaterial applied to mass analysis.

6. Use of a container according to any one of claims 1 to 2 for fragmentation treatment of a protein and/or a peptide.

7. An analytical method of a fragmented protein and/or peptide which comprises
preparing a sample containing a protein and/or a peptide which is/are an analytical object(s),
subjecting the protein and/or the peptide in the sample to fragmentation treatment in a container provided with a coating containing a copolymer which contains a recurring unit which contains a group represented by the following formula (a) and a recurring unit which contains a group represented by the following formula (b): 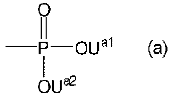 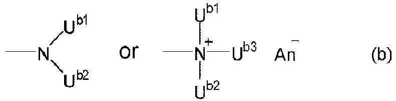
wherein
U^{a1}, U^{a2}, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion,
at least a part of a surface of the container, and
applying the fragmented protein and/or peptide in the sample to a measurement apparatus.

8. A method for measuring an absolute amount(s) of a biomarker(s) in a solution, which comprises
a step of containing and/or storing a solution containing a biomarker(s) contained in a biomaterial in a container for storing the biomaterial having a coating as defined in claim 1
on at least a part of a surface of the container, and then, a step of measuring an absolute amount(s) of the biomarker(s).

9. The method according to Claim 7, wherein the biomarker(s) is/are an antibody or amyloid.

## Patentansprüche

1. Verwendung eines Behälters zum Aufbewahren eines Biomaterials zum Reduzieren des Probenverlusts eines oder mehrerer in dem Biomaterial enthaltener Biomarker, umfassend
eine Beschichtung, die ein Copolymer enthält, das eine Repetiereinheit, die eine durch die folgende Formel (a) dargestellte Gruppe enthält, und eine Repetiereinheit, die eine durch die folgende Formel (b) dargestellte Gruppe enthält, enthält: 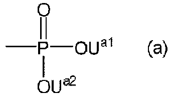 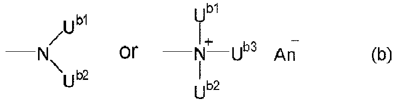
wobei
U^{a1}, U^{a2}, U^{b1}, U^{b2} und U^{b3} jeweils unabhängig für ein Wasserstoffatom oder eine lineare oder verzweigte Alkylgruppe mit 1 bis 5 Kohlenstoffatomen stehen und An⁻ für ein Anion steht, das aus der Gruppe ausgewählt ist, die aus einem Halogenid-Ion, einem Ion einer anorganischen Säure, einem Hydroxid-Ion und einem Isothiocyanat-Ion besteht,
wobei die Beschichtung wenigstens einen Teil einer Oberfläche des Behälters bedeckt.

2. Verwendung gemäß Anspruch 1, wobei das Copolymer eine Repetiereinheit enthält, die eine durch die folgende Formel (c) dargestellte Gruppe enthält:
-R^{c} (c)
wobei
R^{c} für eine lineare oder verzweigte Alkylgruppe mit 1 bis 18 Kohlenstoffatomen, eine cyclische Hydrocarbylgruppe mit 3 bis 10 Kohlenstoffatomen, eine Arylgruppe mit 6 bis 10 Kohlenstoffatomen, eine Aralkylgruppe mit 7 bis 15 Kohlenstoffatomen oder eine Aryloxyalkylgruppe mit 7 bis 15 Kohlenstoffatomen steht, wobei der Arylteil mit einer linearen oder verzweigten Alkylgruppe mit 1 bis 5 Kohlenstoffatomen, die mit einem oder mehreren Halogenatomen substituiert sein kann, substituiert sein kann.

3. Verwendung gemäß Anspruch 1 oder 2, wobei es sich bei dem oder den Biomarkern um einen Antikörper oder ein Amyloid handelt.

4. Verwendung eines Behälters gemäß einem der Ansprüche 1 bis 2 zum Aufbewahren eines Peptids.

5. Verwendung des Behälters zum Aufbewahren eines Peptids gemäß Anspruch 4, wobei das Peptid in einem Biomaterial enthalten ist, das einer Massenanalyse unterzogen wird.

6. Verwendung eines Behälters gemäß einem der Ansprüche 1 bis 2 zur Fragmentierungsbehandlung eines Proteins und/oder eines Peptids.

7. Analyseverfahren für ein fragmentiertes Protein und/oder Peptid, umfassend
das Vorbereiten einer Probe, die ein Protein und/oder ein Peptid enthält, bei denen es sich um analytische Objekte handelt,
das Durchführen einer Fragmentierungsbehandlung mit dem Protein und/oder dem Peptid in der Probe on einem Behälter, der mit einer Beschichtung versehen ist, die ein Copolymer enthält, das eine Repetiereinheit, die eine durch die folgende Formel (a) dargestellte Gruppe enthält, und eine Repetiereinheit, die eine durch die folgende Formel (b) dargestellte Gruppe enthält, enthält: 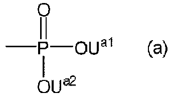 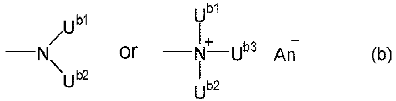
wobei
U^{a1}, U^{a2}, U^{b1}, U^{b2} und U^{b3} jeweils unabhängig für ein Wasserstoffatom oder eine lineare oder verzweigte Alkylgruppe mit 1 bis 5 Kohlenstoffatomen stehen und An⁻ für ein Anion steht, das aus der Gruppe ausgewählt ist, die aus einem Halogenid-Ion, einem Ion einer anorganischen Säure, einem Hydroxid-Ion und einem Isothiocyanat-Ion besteht,
wobei die Beschichtung wenigstens einen Teil einer Oberfläche des Behälters bedeckt, und
Auftragen des fragmentierten Proteins und/oder Peptids in der Probe auf eine Messvorrichtung.

8. Verfahren zum Messen der absoluten Menge eines oder mehrerer Biomarker in einer Lösung, umfassend
einen Schritt des Aufnehmens und/oder Lagerns einer Lösung, die einen oder mehrere Biomarker enthält, die in einem Biomaterial enthalten sind, in einem Behälter zum Aufbewahren des Biomaterials, der auf wenigstens einem Teil einer Oberfläche des Behälters eine Beschichtung aufweist, wie sie in Anspruch 1 definiert ist, und
dann einen Schritt des Messens der absoluten Menge des oder der Biomarker.

9. Verfahren gemäß Anspruch 7, wobei es sich bei dem oder den Biomarkern um einen Antikörper oder ein Amyloid handelt.

## Revendications

1. Utilisation d'un récipient pour stocker un biomatériau pour réduire la perte dans un échantillon d'un ou plusieurs biomarqueurs contenus dans le biomatériau, comprenant
un revêtement contenant un copolymère qui contient un motif récurrent contenant un groupe représenté par la formule (a) suivante, et un motif récurrent contenant un groupe représenté par la formule (b) suivante : 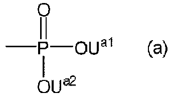 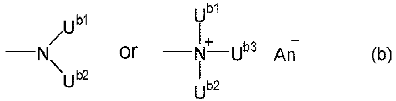
où
U^{a1}, U^{a2}, U^{b1}, U^{b2} et U^{b3} chacun indépendamment représente un atome d'hydrogène, ou un groupe alkyle linéaire ou ramifié ayant 1 à 5 atomes de carbone, et An⁻ représente un anion choisi dans le groupe consistant en un ion halogénure, un ion d'acide inorganique, un ion d'hydroxyde, et un ion d'isothiocyanate,
procuré sur au moins une partie de la surface du récipient.

2. Utilisation selon la revendication 1, dans laquelle ledit copolymère contient un motif récurrent contenant un groupe représenté par la formule (c) suivante :
-R^{c} (c)
où
R^{c} représente un groupe alkyle linéaire ou ramifié ayant 1 à 18 atomes de carbone, un groupe hydrocarbure cyclique ayant 3 à 10 atomes de carbone, un groupe aryle ayant 6 à 10 atomes de carbone, un groupe aralkyle ayant 7 à 15 atomes de carbone, ou un groupe aryloxyalkyle ayant 7 à 15 atomes de carbone, où la partie aryle peut être substituée avec un groupe alkyle linéaire ou ramifié ayant 1 à 5 atomes de carbone, qui peut être substitué avec un ou plusieurs atomes d'halogène.

3. Utilisation selon la revendication 1 ou 2, dans laquelle ledit ou lesdits biomarqueurs sont un anticorps ou un amyloïde.

4. Utilisation d'un récipient selon l'une quelconque des revendications 1 à 2 pour stocker un peptide.

5. Utilisation d'un récipient pour stocker un peptide selon la revendication 4, dans laquelle le peptide est contenu dans un biomatériau appliqué à une analyse de masse.

6. Utilisation d'un récipient selon l'une quelconque des revendications 1 à 2 pour un traitement de fragmentation d'une protéine et/ou d'un peptide.

7. Méthode analytique pour une protéine fragmentée et/ou un peptide fragmenté, comprenant les étapes consistant à
préparer un échantillon contenant une protéine et/ou un peptide qui sont des objets analytiques,
soumettre la protéine et/ou le peptide dans l'échantillon à un traitement de fragmentation dans un récipient, procuré avec un revêtement contenant un copolymère qui contient un motif récurrent contenant un groupe représenté par la formule (a) suivante, et un motif récurrent contenant un groupe représenté par la formule (b) suivante : 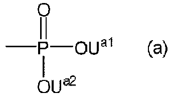 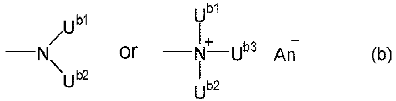
où
U^{a1}, U^{a2}, U^{b1}, U^{b2} et U^{b3} chacun indépendamment représente un atome d'hydrogène, ou un groupe alkyle linéaire ou ramifié ayant 1 à 5 atomes de carbone, et An⁻ représente un anion choisi dans le groupe consistant en un ion halogénure, un ion d'acide inorganique, un ion d'hydroxyde, et un ion d'isothiocyanate,
procuré sur au moins une partie de la surface du récipient, et
appliquer la protéine fragmentée et/ou le peptide fragmenté dans l'échantillon à un dispositif de mesure.

8. Procédé pour mesurer la quantité absolue d'un ou de plusieurs biomarqueurs dans une solution, comprenant
une étape consistant à contenir et/ou stocker une solution contenant un ou plusieurs biomarqueurs contenus dans un biomatériau dans un récipient pour stocker le biomatériau ayant un revêtement tel que défini dans la revendication 1
procuré sur au moins une partie de la surface du récipient, et ensuite une étape consistant à mesurer la quantité absolue dudit ou desdits biomarqueurs.

9. Procédé selon la revendication 7, dans lequel ledit ou lesdits biomarqueurs sont un anticorps ou un amyloïde.
